(19) Europäisches Patentamt
European Patent Office
Office européen des brevets

(11) Veröffentlichungsnummer: **0 456 073 B1**

(12) **EUROPÄISCHE PATENTSCHRIFT**

(45) Veröffentlichungstag der Patentschrift: **25.10.95**

(51) Int. Cl.⁶: **C07C 309/73**, G03F 7/004, C07D 333/34, C07C 309/65, C07D 251/32, C07C 309/67, C07C 309/77, C07D 209/48

(21) Anmeldenummer: **91106918.5**

(22) Anmeldetag: **29.04.91**

Die Akte enthält technische Angaben, die nach dem Eingang der Anmeldung eingereicht wurden und die nicht in dieser Patentschrift enthalten sind.

(54) **Mehrfunktionelle Verbindungen mit alpha-Diazo-beta-ketoester- und Sulfonsäureester-Einheiten und Verfahren zu ihrer Herstellung.**

(30) Priorität: **08.05.90 DE 4014649**

(43) Veröffentlichungstag der Anmeldung:
**13.11.91 Patentblatt 91/46**

(45) Bekanntmachung des Hinweises auf die Patenterteilung:
**25.10.95 Patentblatt 95/43**

(84) Benannte Vertragsstaaten:
**DE FR GB**

(56) Entgegenhaltungen:
**EP-A- 319 325**
**EP-A- 456 075**
**JP-A- 1 265 066**

**PATENT ABSTRACTS OF JAPAN, Band 13, Nr. 478 (P-951)[3826], 30. Oktober 1989; & JP-A-1 188 852 (MATSUSHITA ELECTRIC IND. CO., LTD) 28-07-1989**

(73) Patentinhaber: **HOECHST AKTIENGESELLSCHAFT**

**D-65926 Frankturt am Main (DE)**

(72) Erfinder: **Röschert, Horst, Dr.**
**Am Pfingstborn 16**
**W-6531 Ober-Hilbersheim (DE)**
Erfinder: **Pawlowski, Georg, Dr.**
**Fritz-Kalle-Strasse 34**
**W-6200 Wiesbaden (DE)**
Erfinder: **Merrem, Hans-Joachim, Dr.**
**47 Addison Drive**
**Basking Ridge, NJ 07920 (US)**
Erfinder: **Dammel, Ralph, Dr.**
**Im Ouellborn 16**
**W-6501 Klein-Winternheim (DE)**

**EP 0 456 073 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue mehrfunktionelle Verbindungen mit α-Diazo-β-ketoester- und Sulfonsäureester-Einheiten, ein Verfahren zu ihrer Herstellung und deren Verwendung als photoaktive Komponenten in strahlungsempfindlichen Gemischen.

Photoaktive Diazoderivate enthaltende strahlungsempfindliche Gemische, die für eine Bestrahlung mit energiereicher UV-Strahlung geeignet sind, werden seit einiger Zeit in der Literatur beschrieben.

In der US-A 4 339 522 werden positiv arbeitende strahlungsempfindliche Gemische genannt, die als photoaktive Verbindung ein Diazoderivat der Meldrumsäure enthalten. Diese Verbindung soll für eine Belichtung mit energiereicher UV-Strahlung im Bereich zwischen 200 bis 300 nm geeignet sein. Es zeigte sich aber, daß bei Anwendung dieser Mischungen die photoaktive Verbindung unter den in der Praxis häufig eingesetzten höheren Verarbeitungstemperaturen ausgeschwitzt wird; das strahlungsempfindliche Gemisch verliert seine ursprüngliche Aktivität, so daß reproduzierbare Strukturierungen nicht möglich sind.

In der JP-A 01-265 066, referiert in "Patent Abstracts of Japan, Vol. 14, No. 24 (C-677) [3967]" vom 18. Januar 1990, sind strahlungsempfindliche α-Diazoacetessigsäureester des Pentaerythrits, des Dipentaerythrits, des Cyclohexan-1,4-diols und des Inosits offenbart. Dabei sind sämtliche Hydroxygruppen der genannten Verbindungen mit α-Diazo-acetessigsäure verestert. Die Ester sind empfindlich auch gegenüber Strahlung mit einer Wellenlänge von weniger als 300 nm. Sie sublimieren nicht und zeigen eine gute Löslichkeit.

In den EP-A 0 198 674 und 0 262 864, der JP-A 01-106 034, der US-A 4 622 283 und der SU-A 1 004 359 werden 2-Diazo-cyclohexan-1,3-dion- bzw. -cyclopentan-1,3-dion-Derivateals photoaktive Verbindungen für strahlungsempfindliche Gemische der beschriebenen Art bereitgestellt. Diese Verbindungen weisen eine geringere Flüchtigkeit auf, zeigen aber dafür, je nach vorliegendem Substitutionsmuster, eine schlechte Kompatibilität im strahlungsempfindlichen Gemisch. Dieses äußert sich insbesondere bei Trocknung der Schichten durch ein Auskristallisieren der photoaktiven Verbindung, durch deren Unlöslichkeit in praxisgerechten Lösungsmitteln oder durch eine durch Phasenseparation hervorgerufene Schichtinhomogenität.

Weitere, im tiefen UV-Bereich empfindliche und positiv arbeitende photoaktive Verbindungen sind aus der EP-A 0 129 694 und der US-A 4 735 885 bekannt. Die in diesen Dokumenten beschriebenen Verbindungen zeigen den Nachteil, daß die aus diesen bei Belichtung gebildeten Carbene keine für die gewünschte Carbonsäurebildung ausreichende Stabilität in der Matrix aufweisen. Dies führt zu einem unzureichenden Löslichkeitsunterschied zwischen den belichteten und unbelichteten Bereichen im Entwickler und damit zu einer unerwünscht hohen Abtragsrate der unbelichteten Bereiche. Eine mögliche Erklärung für dieses Phänomen geben C.G. Willson et al. in SPIE Vol. 771, "Advances in Resist Technology and Processing IV", 2 (1987).

In der EP-A 0 195 986 werden daher α-phosphorylsubstituierte Diazocarbonylverbindungen als photoaktive Verbindungen vorgeschlagen, da diese eine erhöhte Carbenstabilität aufweisen. In der Praxis dürften solche Verbindungen jedoch nur geringeren Einsatz finden, da potentiell als Dotiermittel verwendbare Atome, wie beispielsweise der in diesen Verbindungen enthaltene Phosphor, bei den Verarbeitungsprozessen peinlichst ausgeschlossen werden müssen.

H. Sugiyama, K. Ebata, A. Mizushima und K. Nate stellen in ihrem Aufsatz "Positive Excimer Laser Resist Prepared with Aliphatic Diazoketones" [SPIE Proc., 920, 51 (1988)] neue α-Diazoacetoacetate vor, die als photoaktive Verbindungen in positiv arbeitenden strahlungsempfindlichen Gemischen, insbesondere bei Verwendung von Strahlung im tiefen UV-Bereich, eingesetzt werden. Da es sich um Derivate der Acetoessigsäure handelt, ist die zur Estergruppe β-ständige Ketogruppe direkt einer endständigen Methylgruppe benachbart.

Diazoderivate der Acetoessigsäure werden auch in der EP-A 0 319 325 und der DE-A 38 41 571 als strahlungsempfindliche Komponenten im tiefen UV-Bereich (< 300 nm) eingesetzt. Strahlungsempfindliche Gemische, die die genannten Verbindungen als photoaktive Komponenten aufweisen, zeigen gute Ausbleicheigenschaften, ihre Eigenschaften hinsichtlich der Bilddifferenzierung sind aber schlecht.

Aliphatische Bis(carbonyl)diazomethane, die in der JP-A 01-080 944 Verwendung finden, sind durch eine unzureichende thermische Stabilität gekennzeichnet, weshalb solche Verbindungen in der Praxis kaum Verwendung finden dürften. Für Bis(carbonyl)diazomethane, die in den JP-A 63-253 938, 01-106 036 und 01-010 237 beansprucht werden, gilt das gleiche wie für die Derivate der Acetoessigsäure.

Aufgabe der Erfindung war es daher, photoaktive Verbindungen mit hoher Empfindlichkeit im tiefen UV-Bereich bereitzustellen, die die zahlreichen beschriebenen Nachteile nicht aufweisen und die insbesondere als Komponenten in strahlungsempfindlichen Gemischen Schichten ergeben, die eine gute Differenzierung zwischen den belichteten und unbelichteten Schichtbereichen zulassen. Darüber hinaus sollen die Verbindungen mit den unterschiedlichsten, in der Praxis verwendbaren Polymeren gut verträglich sein, aus dem

strahlungsempfindlichen Gemisch nicht ausschwitzen und eine hohe thermische Stabilität sowie praxisgerechte Lichtempfindlichkeiten aufweisen.

Die Lösung der Aufgabe erfolgt durch Bereitstellen mehrfunktioneller Verbindungen mit α-Diazo-β-ketoester- und Sulfonsäureester-Einheiten gemäß der allgemeinen Formel I

$[R^1\text{-CO-C}(N_2)\text{-CO-O}]_{m-n}\text{-X-}[\text{O-SO}_2\text{-}R^2]_n$ (I)

worin

- $R^1$ Ethyl, Isopropyl, ein gegebenenfalls substituierter aliphatischer Rest mit 4 bis 20 Kohlenstoffatomen oder ein gegebenenfalls substituierter cycloaliphatischer, araliphatischer oder aromatischer Rest mit 4 bis 20 Kohlenstoffatomen ist, in denen jeweils einzelne $CH_2$-Gruppen durch Sauerstoff- oder Schwefelatome, NH- und/oder CO-Gruppen ersetzt sein können und einzelne CH-Gruppen in einer aliphatischen Kette durch -N< ersetzt sein können,
- $R^2$ ein gegebenenfalls substituierter aliphatischer Rest mit 1 bis 20 Kohlenstoffatomen oder ein gegebenenfalls substituierter cycloaliphatischer, araliphatischer oder aromatischer Rest mit 4 bis 20 Kohlenstoffatomen ist, in denen jeweils einzelne $CH_2$-Gruppen durch Sauerstoff- oder Schwefelatome, NH- und/oder CO-Gruppen und einzelne CH-Gruppen durch -N< ersetzt sein können,
- X ein gegebenenfalls substituierter aliphatischer, cycloaliphatischer, carbocyclischer, heterocyclischer oder araliphatischer Rest mit 2 bis 22 Kohlenstoffatomen ist, in dem einzelne $CH_2$-Gruppen durch Sauerstoff- oder Schwefelatome, $-NR^3-$ oder CO-Gruppen und einzelne CH-Gruppen durch -N< ersetzt sein können, worin
- $R^3$ für Wasserstoff oder einen gegebenenfalls substituierten aliphatischen, carbocyclischen oder araliphatischen Rest steht,
- m eine ganze Zahl von 3 bis 8 und
- n eine ganze Zahl von 1 bis 3 ist, wobei
- m-n $\geqq$ 2 ist.

$R^3$ bedeutet in der bevorzugten Ausführungsform Wasserstoff, $(C_1\text{-}C_3)$Alkyl, $(C_6\text{-}C_{12})$Aryl oder $(C_6\text{-}C_{11})$-Aralkyl, wobei Aryl und Aralkyl gegebenenfalls am Kern durch $(C_1\text{-}C_3)$Alkyl, $(C_1\text{-}C_3)$Alkoxy, Halogen, insbesondere Chlor oder Brom, oder Amino substituiert sein können.

Die für $R^1$, $R^2$ und X stehenden Reste können ggf. substituiert sein, insbesondere durch $(C_1\text{-}C_3)$Alkyl, $(C_1\text{-}C_3)$Alkoxy, Halogen, insbesondere Chlor oder Brom, Nitril, Amino oder Nitro. Bevorzugt sind Reste $R^1$, $R^2$ und X, die substituiert sind durch $(C_1\text{-}C_3)$Alkyl oder $(C_1\text{-}C_3)$Alkoxy. Insbesondere wenn $R^1$ und X für einen Alkyl- bzw. Alkylenrest stehen, werden die unsubstituierten Derivate bevorzugt. Wenn $R^2$ ein Alkylrest bedeutet, werden dagegen die substituierten Derivate bevorzugt.

Die aliphatischen Reste $R^1$ können sowohl geradkettig als auch verzweigt sein. Die Zahl der Kettenglieder beträgt hierbei bevorzugt 4 bis 10, insbesondere 4 bis 8. Hierunter fallen die besonders bevorzugten reinen Kohlenstoffketten wie auch die substituierten, in denen $CH_2$-Gruppen durch Sauerstoffatome oder -NH-Gruppen ersetzt und/oder Ketogruppen enthalten sind, worunter auch Ether-, Keto-, Ester-, Amido- oder Imidogruppen zu sehen sind, d.h. auch Ester der Carbaminsäure. In den reinen aliphatischen Resten $R^1$ treten Ethergruppen besonders bevorzugt zweimal pro Rest $R^1$ auf. Sofern es sich um reine, insbesondere geradkettige, Kohlenstoffketten handelt, ist eine Begrenzung der Kohlenstoffzahl nicht zwingend; es können durchaus aliphatische Reste mit bis zu 20 Kohlenstoffatomen zum Einsatz kommen. Besonders bevorzugt ist dennoch der tert.-Butylrest.

Sofern $R^1$ ein cycloaliphatischer Rest bedeutet, ist die Zahl der Ringglieder bevorzugt 4, 5, 6 oder 12, insbesondere 4, 5 oder 6. Die unsubstituierten Varianten werden besonders bevorzugt. Als Beispiele gelten der Cyclobutyl-, Cyclopentyl- und der Cyclohexylrest. Besonders bevorzugt ist der Cyclohexylrest.

Ist $R^1$ ein araliphatischer Rest, so ist die Zahl der Glieder des aliphatischen Teils des Restes 2 bis 11, insbesondere 2 bis 5. Sofern es sich um eine reine Kohlenstoffkette im aliphatischen Teil handelt, ist die Anzahl der C-Atome bevorzugt 1 oder 2. Werden $CH_2$-Gruppen durch Sauerstoffatome ersetzt, so können diese als Brückenglieder zwischen aromatischem und aliphatischem Teil des Rests $R^1$ vorkommen, aber auch im aliphatischen Teil selbst. In beiden Fällen ist es besonders bevorzugt, wenn die verbleibende Gesamtzahl der Kohlenstoffatome als Kettenglieder im aliphatischen Teil dieses Restes 1 oder 2 beträgt, wobei das Ether-Sauerstoffatom im Falle von 2 Kohlenstoffatomen als Kettenglieder in der Weise angeordnet ist, daß es den beiden $CH_2$-Gruppen direkt benachbart ist. Hierunter sind zu nennen der Benzyl-, der Phenoxymethyl- und der Benzyloxymethylrest. Werden zusätzlich noch weitere $CH_2$-Gruppen im aliphatischen Teil des araliphatischen Restes $R^1$ durch Heteroatome ersetzt und/oder Substitutionen an diesem vorgenommen, so ist die Gesamtzahl der Kettenglieder des aliphatischen Teils 3 bis 5. Hierunter fallen u.a.

über Estergruppen gebundene Phenyl- oder Benzylreste, aber auch die Benzyl- oder Phenylester der Carbaminsäure. Der aliphatische Teil kann aber auch die Imidogruppe einer aromatischen Dicarbonsäure darstellen. Der aromatische Teil eines solchen Restes besteht insbesondere aus 6 C-Atomen. Wenn der aromatische Teil des araliphatischen Restes direkt benachbart zur Ketogruppe steht, d.h. als Arylenrest eingebunden ist, so gelten für den darin vorkommenden aliphatischen Teil keine Beschränkungen hinsichtlich der Zahl mindestens vorkommender C-Atome.

Die aromatischen Reste $R^1$ weisen bevorzugt keine Heteroatome wie z.B. Sauerstoff im Ringsystem auf. Ist $R^1$ ein aromatischer Rest, so enthält er bevorzugt 6 bis 12 C-Atome, insbesondere 6 C-Atome, d.h. er entspricht einem Phenylrest. Aromatische Reste $R^1$ sind generell aber nicht bevorzugt.

Insgesamt sind als besonders bevorzugte Reste $R^1$ t-Butyl, n-Hexyl, Nonyl, Octadecyl, 2,5-Dioxahexyl, Cyclopentyl, Cyclohexyl, Benzyl, Phenoxymethyl und Benzyloxymethyl zu nennen. Insbesondere bevorzugt ist der t-Butyl-, Phenoxymethyl- und der Cyclohexylrest.

Die aliphatischen Reste $R^2$ können sowohl geradkettig als auch verzweigt sein. Die Zahl der Kettenglieder beträgt hierbei bevorzugt 1 bis 8, insbesondere 2 bis 6. Hierunter fallen sowohl reine Kohlenstoffketten als auch die substituierten, in denen $CH_2$-Gruppen durch Sauerstoffatome oder -NH-Gruppen ersetzt und/oder Ketogruppen enthalten sind, worunter Ether-, Keto-, Ester-, Amido oder Imidogruppen fallen. In den reinen aliphatischen Resten $R^2$ treten verzweigte Kohlenstoffketten bevorzugt auf. Eine Begrenzung der Kohlenstoffzahl ist nicht zwingend; es können durchaus aliphatische Reste mit mehr als 8 Kohlenstoffatomen auftreten. Bevorzugt sind dennoch der i-Propyl- und der t-Butylrest.

Sofern $R^2$ ein cycloaliphatischer Rest bedeutet, ist die Zahl der Ringglieder bevorzugt 4, 5, 6 oder 12, insbesondere 5 oder 6. Die unsubstituierten Varianten werden bevorzugt. Cycloaliphatische Reste $R^2$ sind aber insgesamt nicht bevorzugt.

Ist $R^2$ ein araliphatischer Rest, so ist die Zahl der Glieder des aliphatischen Teils 2 bis 11, insbesondere 2 bis 5. Sofern es sich um eine reine Kohlenstoffkette handelt, ist die Anzahl der C-Atome bevorzugt 1. Werden $CH_2$-Gruppen durch Sauerstoffatome ersetzt, so können diese als Brückenglied zwischen aromatischem und aliphatischem Teil des Rests $R^2$ vorkommen, aber auch im aliphatischen Teil. In beiden Fällen ist es besonders bevorzugt, wenn die verbleibende Gesamtzahl der Kohlenstoffatome als Kettenglieder im aliphatischen Teil dieses Restes 1 oder 2 beträgt. Der aromatische Teil des araliphatischen Restes $R^2$ besteht insbesondere aus 6 C-Atomen.

Die aromatischen Reste $R^2$ können Heteroatome, wie Schwefel oder Sauerstoff, in ihrem Ringsystem aufweisen. Diese sind bevorzugt substituiert. Ist $R^2$ ein aromatischer Rest, so enthält er bevorzugt 6 bis 12 C-Atome, insbesondere 6 C-Atome, d.h. er entspricht einem Phenylrest. Aromatische Reste $R^2$ sind besonders bevorzugt. Sind die aromatischen Reste $R^2$ substituiert, so handelt es sich bevorzugt um Alkyl-, Alkoxy-, Halogen-, Nitril- und Nitrogruppen. Aber auch Amino- und Amidogruppen kommen als Substituenten in Frage. Hinsichtlich der Anzahl der Substituenten und des Substitutionsmusters gelten keine Beschränkungen.

Insgesamt sind als besonders bevorzugte Reste $R^2$ niedere Alkylreste ($C_1$-$C_4$) wie i-Propyl, t-Butyl, die ggf. mit Halogenatomen substituiert sind, und aromatische Verbindungen zu nennen. Insbesondere bevorzugt sind in 4-Stellung mit Methyl-, t-Butyl-, Trifluormethyl-, Brom- oder Cyanogruppen substituierte Phenylreste.

X steht für einen aliphatischen oder cycloaliphatischen Rest, der gesättigt wie auch ungesättigt sein kann, einen carbocyclischen, heterocyclischen oder araliphatischen Rest mit 2 bis 22 Kohlenstoffatomen, insbesondere mit 2 bis 17 Kohlenstoffatomen. In diesen Resten kann zudem mindestens eine $CH_2$-Gruppe durch Heteroatome wie Sauerstoff, Schwefel oder durch Gruppen wie $-NR^3-$, -C(O)-O-, $-C(O)-NR^3-$,

$$-C(O)-\underset{|}{N}-,$$

$-NR^3-C(O)-NR^4-$, $-O-C(O)-NR^3-$,

$$-O-C(O)-\underset{|}{N}-$$

oder -O-C(O)-O- oder CH-Gruppen durch

$$-\underset{|}{N}-$$

ersetzt sein. Insbesondere bevorzugt sind bei den aliphatischen oder araliphatischen Resten diejenigen Varianten, in denen maximal zwei $CH_2$-Gruppen durch einen Typ der oben genannten Gruppen ersetzt worden sind. Werden CH2-Gruppen durch Heteroatome ersetzt, so kann die Anzahl dieser bevorzugt maximal 5, insbesondere maximal 3 sein. Dabei ist besonders bevorzugt, wenn alle zu ersetzenden CH2-Gruppen durch Heteroatome eines Typs ersetzt werden.

Handelt es sich um einen unsubstituierten, gesättigten oder ungesättigten aliphatischen Rest X, so enthält dieser in der bevorzugten Variante maximal 6 Kohlenstoffatome. Zu den ungesättigten aliphatischen Resten X zählen insbesondere solche, deren $CH_2$- bzw. CH-Gruppen nicht durch Heteroatome bzw. den obengenannten Gruppen ersetzt sind. Sie enthalten in ihrer besonderen Ausführungsform maximal eine C-C-Mehrfachbindung; die Zahl der Kettenglieder ist in einem solchen Rest besonders bevorzugt 4. Die genannten Reste X sind bevorzugt 3-bindig.

Werden in den aliphatischen Resten X $CH_2$-Gruppen durch Heteroatome ersetzt, so werden diese bevorzugt von Alkylenresten mit jeweils mindestens 2 $CH_2$-Gruppen flankiert. Ist Schwefel das Heteroatom im aliphatischen Rest X, so kommt dieser besonders bevorzugt pro Rest lediglich einmal vor. Er ist insbesondere von Alkylenresten mit jeweils maximal 3 $CH_2$-Gruppen umgeben. Wird Sauerstoff als Heteroatom im aliphatischen Rest eingesetzt, so kann dieser häufiger pro Rest vorkommen, insbesondere 2 bis 4 mal. In diesem Fall enthalten die Alkylenreste, in die zwei Sauerstoffatome eingebunden sind, mindestens 3 $CH_2$-Gruppen.

Ist die Anzahl der Kohlenstoffatome im unsubstituierten aliphatischen Rest größer als 3, so liegt dieser Alkylenrest insbesondere als verzweigtes Isomeres vor. Insbesondere bevorzugt ist der t-Butyl- oder der t-Pentylrest.

Im Rest X können auch mehrere Alkylreste, insbesondere der t-Butyl- oder t-Pentylrest, über Heteroatome oder $CH_2$-Gruppen ersetzende Gruppen, die oben genannt sind, verbunden sein. Dies ist dann besonders bevorzugt, wenn diese Reste mehr als zweibindig sind:

Sofern die t-Butyl oder t-Pentylreste dreibindig sind, ist m mindestens 3. m = 4 wird bevorzugt erreicht, indem ein zweibindiger t-Pentyl- oder t-Butylrest im Rest X zweimal vorliegt. m = 4 wird auch erreicht, indem ein zweibindiger Propylrest am Rest X zweimal vorkommt.

m = 6 wird bevorzugt erreicht, indem zwei dreibindige Reste (t-Butyl oder t-Pentyl) oder drei zweibindige Propylreste im Rest X vorliegen. m = 8 wird z.B. erreicht durch die Kombination von 4 zweibindigen Resten oder von 2 dreibindingen Resten mit einem zweibindigen Rest.

Die mehr als Zweibindigkeit des Restes X kann auch dadurch erreicht werden, daß mehr als zweibindige Heteroatome enthalten sind: Wird im aliphatischen Rest X eine CH-Gruppe durch

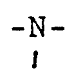

ersetzt, so können m-Werte von 3 erreicht werden. Werden zwei CH-Gruppen durch

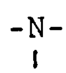

ersetzt, so kann m maximal 4 erreichen.

In allen Fällen, in denen im Rest X CH-Gruppen durch

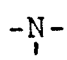

ersetzt werden, findet bevorzugt keine Substitution einer $CH_2$-Gruppe durch ein weiteres Heteroatom oder durch eine der oben beschriebenen Gruppen statt. Die Anzahl der $CH_2$-Gruppen, die zwischen dem Stickstoffheteroatom und dem an den Rest X gebundenen Rest nach der allgemeinen Formel I liegen, beträgt mindestens 2; insbesondere ist diese die Zahl 2.

Reine cycloaliphatische Reste, d.h. unsubstituierte, als Varianten des Restes X sind nicht bevorzugt. Als cycloaliphatischer Rest wird insbesondere der Cyclohexylrest genannt. Dieser kann aber substituiert sein, insbesondere durch Hydroxyl und/oder Alkyl oder Alkylen, wobei dessen Bindigkeit bevorzugt durch die Anzahl der Alkylensubstituenten am cycloaliphatischen Rest bestimmt wird. Ganz besonders bevorzugt ist ein Cyclohexylrest, der vier Methylengruppen als Substituenten tragt, die die Bindung der $\alpha$-Diazo-$\beta$-

ketoester- und der Sulfonsäureester-Einheiten nach der allgemeinen Formel I gewährleisten (m = 5).

Zumeist handelt es sich bei den cycloaliphatischen Resten als Varianten des Restes X mehr um eine Kombination aus einem cycloaliphatischen und einem kettenförmigen aliphatischen Teil. In diesem Fall ist der cycloaliphatische Teil bevorzugt nicht in der Weise substituiert, daß $CH_2$-Gruppen aus diesem Teil durch Heteroatome oder durch Gruppen aus der obengenannten Reihe ersetzt sind.

Als Ausnahme gilt ein sechsgliedriger Ring (Heterocyclus) aus drei Carbonsäureamideinheiten, in dem die Koppelung der α-Diazo-β-ketoester- und der Sulfonsäureester-Einheiten der allgemeinen Formel I über Ethylengruppen an den Amidostickstoff erfolgt. m ist daher 3 und n ist 1.

Wenn X aber eine Kombination aus einem reinen cycloaliphatischen Teil und einem oder mehreren kettenförmigen aliphatischen Teilen mit 2 oder mehreren Kohlenstoffatomen ist, so ist der cycloaliphatische Teil insbesondere einer $CH_2$-Gruppe, die durch eine der obengenannten Heteroatome oder Gruppen ersetzt ist, direkt benachbart. Besonders bevorzugt ist diejenige Variante, in der der cycloaliphatische Teil einem Stickstoffatom, insbesondere den Gruppen

$$-NH-\overset{O}{\overset{\|}{C}}-O- \quad \text{und} \quad -NH-\overset{O}{\overset{\|}{C}}-NH-,$$

direkt benachbart ist. In diesem Fall wird als cycloaliphatischer Teil bevorzugt ein Cyclohexylrest eingesetzt, der sowohl einbindig als auch zweibindig sein kann, letzteres bevorzugt in 1,4-Position. In beiden Fällen erfolgt die Verknüpfung einer der freien Valenzen der Reste

$$-NH-\overset{O}{\overset{\|}{C}}-O- \quad \text{oder} \quad -NH-\overset{O}{\overset{\|}{C}}-NH-$$

mit α-Diazo-β-ketoester- und Sulfonsäureester-Einheiten nach der allgemeinen Formel I über ein Alkylenrest mit mindestens 2 $CH_2$-Einheiten.

Sofern es sich um einen

$$-NH-\overset{O}{\overset{\|}{C}}-NH\text{-Rest}$$

handelt, ist als verknüpfende Gruppe bevorzugt ein t-Butylenrest formuliert. Sofern es sich um einen

$$-NH-\overset{O}{\overset{\|}{C}}-O\text{-Rest}$$

handelt, d.h. die diskutierte Verknüpfung über das Sauerstoffatom dieser Gruppe erfolgt, wird bevorzugt ein Ethylenrest als verknüpfende Gruppe eingesetzt.

Handelt es sich um einen araliphatischen Rest als Variante der Gruppe X, so kann der aromatische Teil, insbesondere ein Phenyl-, bzw. wenn Zweibindigkeit vorliegt, ein Phenylenrest, sowohl über ein Stickstoffatom als auch über ein Sauerstoffatom gebunden sein. Bevorzugt ist allerdings auch hier - wenn beide Atome zur Auswahl stehen - das Stickstoffatom. Als Beispiel, in dem der aromatische Teil direkt über ein Sauerstoffatom gebunden ist, zählt ein über eine Ethylengruppe an die α-Diazo-β-ketoester- und/oder Sulfonsäureester-Einheit gebundenes Ethersauerstoffatom. Als dritte Variante ergibt sich in diesem Fall noch die Möglichkeit, daß der aromatische Rest, insbesondere wenn er einbindig ist, über die Ketogruppe eines Restes

$$-\overset{O}{\overset{\|}{C}}-NH-$$

gebunden vorliegt. Das Stickstoffatom trägt in diesem Fall insbesondere einen Ethylenrest.

Im Falle des Vorliegens eines araliphatischen Restes X ist der über das Stickstoffatom eines eine $CH_2$-Gruppe ersetzenden Restes

$$-NH-\overset{O}{\overset{\|}{C}}-NH- \quad \text{oder} \quad -O-\overset{O}{\overset{\|}{C}}-NH-$$

gebundene aliphatische Teil des Restes X insbesondere ein t-Butylen- oder ein Ethylenrest; ist der aliphatische Teil über das Sauerstoffatom gebunden, so ist dieser besonders bevorzugt ein Ethylenrest.

Daß über das Sauerstoffatom der obengenannten, eine $CH_2$-Gruppe im Rest X zu ersetzenden Gruppen bevorzugt eine Ethylengruppe gebunden ist, läßt sich auch auf aliphatische Reste im allgemeinen übertragen, während über das Stickstoffatom sowohl Ethylenreste wie auch höhere aliphatische Reste, insbesondere aus Kohlenstoffketten mit mehr als 3 Kohlenstoffatomen, gebunden sind. Bevorzugt ist der t-Butylenrest.

Sofern es sich um araliphatische und aliphatische Reste X handelt, werden bevorzugt maximal 2 $CH_2$-Gruppen durch Reste wie

$$-NH-\overset{O}{\overset{\|}{C}}-NH- \quad \text{oder} \quad -O-\overset{O}{\overset{\|}{C}}-NH-$$

ersetzt. Der Rest

$$-\overset{O}{\overset{\|}{C}}-\underset{|}{N}-$$

als Ersatz für eine CH-Gruppe liegt in der besonders bevorzugten Ausführungsform nur einmal in einem Rest X vor.

In den oben dargestellten Varianten der Reste $R^1$, $R^2$ und X ist m bevorzugt eine ganze Zahl von 3 bis 8 und n eine ganze Zahl von 1 bis 3.

Besonders bevorzugt ist m eine ganze Zahl von 3 bis 6 und n = 1 oder 2.

Die vorstehend näher charakterisierten, erfindungsgemäßen mehrfunktionellen Verbindungen der allgemeinen Formel I sind als photoaktive Komponenten in einem strahlungsempfindlichen Gemisch hervorragend geeignet. Insbesondere sind die erfindungsgemäßen Verbindungen geeignet für die Belichtung mit Strahlung einer Wellenlänge von 190 bis 350 nm. Die Verwendung der neuen mehrfunktionellen Verbindungen als photoaktive Komponenten in strahlungsempfindlichen Gemischen zur Herstellung von hochauflösenden Photoresists für die Mikrolithographie wird in der gleichzeitig eingereichten deutschen Patentanmeldung P 40 14 648.0 beschrieben.

Ebenso ist Gegenstand der Erfindung ein Verfahren zur Herstellung der erfindungsgemäßen, mehrfunktionellen Verbindungen mit $\alpha$-Diazo-$\beta$-ketoester- und Sulfonsäureester-Einheiten. Es hat sich als besonders günstig erwiesen, zunächst geeignete Vorstufen für $\beta$-Ketoester-Sulfonsäureester der allgemeinen Formel II zu synthetisieren und diese in einer Folgereaktion durch einen sogenannten Diazotransfer in die $\alpha$-Diazo-$\beta$-ketoester-Sulfonsäureester der Formel I zu überführen (vgl. M. Regitz et al., Org. Prep. Proced., 1, 99 (1969)):

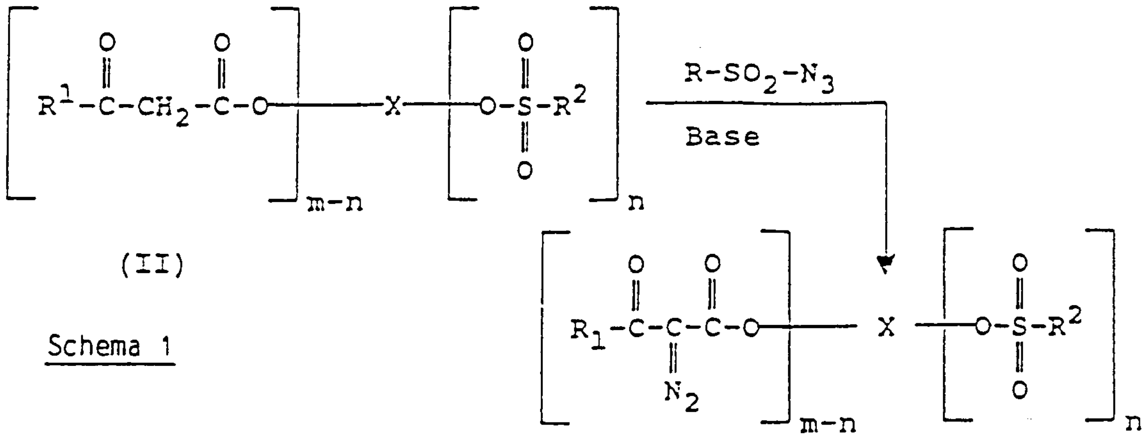


(II)

Schema 1

(I)

Zu diesem Zweck wird ein $\beta$-Ketoester-Sulfonsäureester der allgemeinen Formel II (in der $R^1$, $R^2$ und X die in Formel I angegebene Bedeutung aufweisen) in der 5 bis 50fachen, bevorzugt 10fachen Menge eines geeigneten Lösungsmittels (bezogen auf das Gemisch) gelöst und die Lösung auf eine Temperatur zwischen -15 °C bis +15 °C, bevorzugt -5 °C bis +5 °C gekühlt. Als Lösungsmittel geeignet sind Alkohole, wie Methanol, Ethanol, Alkoholether wie Ethylenglykolmonomethylether, chlorierte Kohlenwasserstoffe, wie Dichlormethan oder Trichlormethan, oder bevorzugt aliphatische Nitrile, wie Acetonitril, oder Gemische dieser Lösungsmittel. Besonders bevorzugt werden dabei solche, die einen Siedepunkt zwischen 30 °C und 140 °C aufweisen. Die eigentliche erfindungsgemäße Umsetzung kann nach 3 Varianten durchgeführt werden.

Variante A:

Die gekühlte Lösung wird mit dem 1- bis 1,3-fachen Überschuß - bezogen auf die Anzahl der umzusetzenden aktivierten Methylengruppen - eines Diazotransferreagenzes versetzt. Als Transferreagenzien haben sich insbesondere aromatische und aliphatische Sulfonylazide, wie Toluolsulfonylazid, 4-Carboxyphenylsulfonylazid, 2-Naphthalinsulfonylazid oder Methylsinlfonylazid bewährt. Bezogen auf das Sulfonylazid wird dann die äquimolare Menge einer Base, bevorzugt eines tertiären Amins, zu der Lösung hinzugefügt. Die Temperatur der Mischung muß dabei konstant gehalten werden. Bevorzugte Amine sind beispielsweise Triethylamin, Triisopropylamin oder Diazo-bicyclo[2.2.2]octan. Besonders bevorzugt ist die Verwendung von Triethylamin als Base. Die erhaltene Mischung wird 5 bis 50 Minuten, bevorzugt 10 bis 15 Minuten, bei der vorgegebenen Temperatur gerührt, auf Raumtemperatur erwärmt und dabei weitere 1 bis 24 Stunden, bevorzugt 2 bis 4 Stunden, gerührt. Je nach Art des eingesetzten Sulfonylazids kann das dabei gebildete Sulfonylamid ausfallen, so daß es nach Beendigung der Reaktion ggf. abfiltiert wird.

Variante B:

Alternativ zu Variante A können auch der $\beta$-Ketoester-Sulfonsäureester der allgemeinen Formel II und das Amin unter den vorstehend beschriebenen Bedingungen vorgelegt werden. Anschließend kann das Sulfonylazid unter Beibehaltung der Temperatur zudosiert werden.

Variante C:

Als besonders günstig hat sich jedoch eine modifizierte Variante A erwiesen, in der die Lösung des $\beta$-Ketoester-Sulfonsäureesters der allgemeinen Formel II zunächst nur mit dem 0,7- bis 0,9fachen Überschuß - bezogen auf die Anzahl der umzusetzenden aktivierten Methylengruppen - eines Sulfonylazids, bevorzugt Toluolsulfonylazid, versetzt wird und unter Beibehaltung der vorgegebenen Temperatur die Gesamtmenge des Amins zugesetzt wird. Die Mischung wird dann ggf. unter Erwärmung auf Raumtemperatur gerührt. Nach etwa 10 bis 120 Minuten ist das Toluolsulfonylazid chromatographisch nicht mehr nachweisbar. Hierauf wird die Mischung ggf. erneut gekühlt und diesmal mit dem 0,6- bis 0,1fachen Überschuß von 4-Carboxyphenylsulfonylazid versetzt, so daß ein Gesamtüberschuß an Sulfonylazid entsprechend der Variante A entsteht. Die Rohprodukte, die nach dieser Variante hergestellt werden, zeigen eine hohe Reinheit.

Das nach den Varianten A bis C erhaltene Gemisch wird vom Lösungsmittel und überschüssigen Reagenzien befreit und in einem inerten, nicht mit Wasser mischbaren Lösungsmittel, insbesondere Dichlormethan oder Diethylether, aufgenommen. Die Mischung wird zur Entfernung von Sulfonylamidresten zweimal mit 5- bis 25%iger Kalilauge und anschließend mit Wasser neutral gewaschen, über einem geeigneten Trocknungsmittel getrocknet und erneut vom Lösungsmittel befreit. Der verbleibende Rückstand, insbesondere bei Anwendung der Variante C fast ausschließlich aus reinem α-Diazo-β-ketoester-Sulfonsäureester der allgemeinen Formel I bestehend, kann nach bekannten Methoden, beispielsweise mittels Kristallisation oder Chromatographie, aufgearbeitet werden.

Die Herstellung der zur Umsetzung zu den erfindungsgemäßen mehrfunktionellen Verbindungen mit α-Diazo-β-ketoester- und Sulfonsäureester-Einheiten der allgemeinen Formel I erforderlichen β-Ketoester-Sulfonsäureester der allgemeinen Formel II kann auf unterschiedliche, aus der Literatur bekannte Verfahrensweisen erfolgen:

1. Entsprechend dem Schema 2 erfolgt eine Umsetzung eines monofunktionellen 5-Acyl-2,2-dialkyl-1,3-dioxan-4,6-dions (5-Acyl-meldrumsäure) der allgemeinen Formel III mit einem mehrwertigen Alkohol-Sulfonsäureester der allgemeinen Formel IV zum mehrfunktionellen β-Ketoester-Sulfonsäurester der allgemeinen Formel II. Die Herstellung von 5-Acyl-meldrumsäurederivaten der allgemeinen Formel III und deren Umsetzung zu β-Ketoestern ist für monofunktionelle Verbindungen bekannt und kann beispielsweise analog zu den Vorschriften von Y. Oikawa et al., J. Org. Chem., 43, 2087 (1987) durch Umsetzung von Säurechloriden mit Meldrumsäure oder analog zu Vorschriften von P. Houghton und D.J. Lapham, Synthesis, 1982, 451 ff., erfolgen. Auch für die Umsetzung zu mehrfunktionellen β-Ketoestern gemäß der obengenannten Literatur findet man Beispiele in den nicht vorveröffentlichten deutschen Patentanmeldungen P 39 00 735.9 und P 39 00 736.7. Die Produkte werden in ihrer Enolform isoliert.

Schema 2

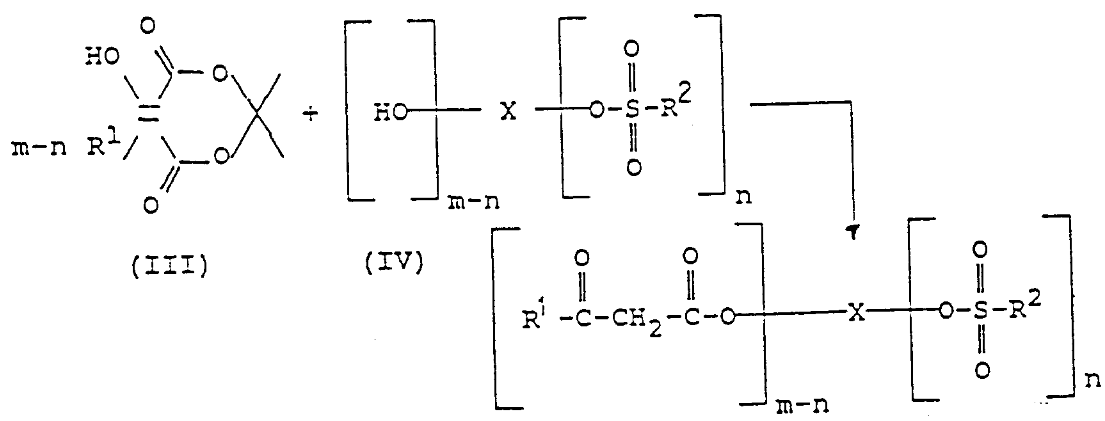

(III) (IV) (II)

2. Entsprechend dem Schema 3 erfolgt die Umsetzung eines monofunktionellen β-Ketoesters, bevorzugt eines Methyl- oder Ethylesters der allgemeinen Formel V, mit einem mehrwertigen Alkohol/Sulfonsäurester der allgemeinen Formel IV zum mehrfunktionellen β-Ketoester der allgemeinen Formel II. Die Umesterungsreaktion zur Herstellung von monofunktionellen β-Ketoestern ist bekannt und wird von A.R. Bader et al. in J. Am. Chem. Soc., 73, 4195 ff., (1951) beschrieben.

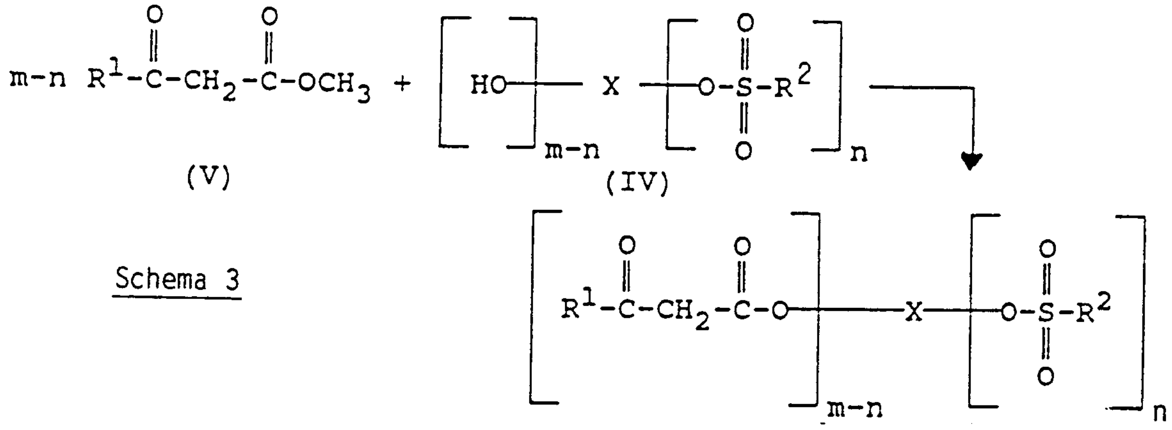

(V) (IV) (II)

Schema 3

Bei der Reaktionsfolge nach Schema 2 wird das betreffende Derivat der 5-Acyl-meldrumsäure der allgemeinen Formel III mit der zum gewünschten Umsetzungsgrad erforderlichen Menge des betreffenden mehrfunktionellen Alkohol-Sulfonsäureesters der allgemeinen Formel IV versetzt und das Gemisch anschließend in der 5- bis 20fachen, bevorzugt 10fachen, Menge eines nicht mit Alkoholen oder der Meldrumsäure reagierenden Lösungsmittels, beispielsweise eines Ketons wie Aceton oder Ethylmethylketon, oder eines Ethers, wie 1,2-Dimethoxyethan oder Dioxan, ggf. unter Erwärmung, gelöst. Die klare Lösung wird auf eine Temperatur von 60 bis 120 °C, bevorzugt auf 80 bis 100 °C, erwärmt. Die beginnende Umsetzung macht sich durch lebhafte Kohlendioxidentwicklung bemerkbar. Die Mischung wird etwa 1 bis 6 Stunden, bevorzugt 2 bis 3 Stunden, bei der vorgegebenen Temperatur gerührt, bis keine $CO_2$-Entwicklung mehr beobachtbar ist.

Anschließend wird das Lösungsmittel im Vakuum abgetrennt. Obwohl der betreffende $\beta$-Ketoester-Sulfonsäureester der allgemeinen Formel II in hoher Reinheit anfällt, kann das Produkt ggf. weiterhin nach den dem Fachmann bekannten Methoden gereinigt werden.

Besonders geeignete Derivate der 5-Acyl-meldrumsäure der allgemeinen Formel III sind solche, in denen $R^1$ Cyclobutyl, Butyl, Pentyl, Cyclopentyl, Hexyl, Cyclohexyl, Heptyl, Octyl, Nonyl, Adamantyl oder höhere Alkylreste mit bis etwa 22 Kohlenstoffatomen bedeuten, die ggf. durch weitere Alkylreste, Alkoxyalkylreste, Arylreste, Alkoxyarylreste, Aryloxyarylreste, Halogenatome oder durch andere mehrfunktionelle Gruppen, beispielsweise durch endständige Säureesterfunktionen, substituiert sind oder in denen einzelne $CH_2$-Gruppen durch Sauerstoff- oder Schwefelatome, durch Gruppen wie -C(O)-O-, -C(O)-$NR^3$-, -$NR^3$-C(O)-$NR^4$-, -O-C(O)-$NR^3$-, -O-C(O)-O-, -$NR^3$-, worin $R^3$ und $R^4$ die vorstehend beschriebene Bedeutung haben, ersetzt sein können.

Als besonders bevorzugte Reste $R^1$ sind 2-Phenylethyl, t-Butyl, n-Hexyl, 2,5-Dioxahexyl, Cyclopentyl, Cyclohexyl, Benzyl, Phenoxymethyl und Nonyl genannt. Insbesondere bevorzugt sind der t-Butyl-, Cyclohexyl- und der Phenoxymethylrest.

Die Herstellung der zur Umsetzung gemäß Schema 2 erforderlichen Alkohol-Sulfonsäureester der allgemeinen Formel IV kann auf unterschiedliche, in der Literatur bekannte, Verfahrensweisen erfolgen:

1) Entsprechend Schema 4 erfolgt die Umsetzung der mehrfunktionellen Alkohole der allgemeinen Formel VI mit entsprechenden Reagenzien unter Ausbildung partiell geschützer Alkohole der allgemeinen Formel VII. Die noch freie(n) Hydroxylgruppe(n) werden mit Sulfonsäurechloriden (Sulfonylchloriden) der allgemeinen Formel VIII unter Einfluß von Base zu den geschützten Alkohol-Sulfonsäureestern der allgemeinen Formel IX umgesetzt. Vollständige Entfernung der vorhandenen Schutzgruppen führt zu den mehrfunktionellen Alkohol-Sulfonsäureestern der allgemeinen Formel IV. Die Schutzgruppentechnik beschreibt T. W. Greene in "Protective Groups in Organic Synthesis", 1. Aufl., S. 10 - 113, Wiley & Sons, New York 1981, ausführlich anhand verschiedener Beispiele.

Schema 4

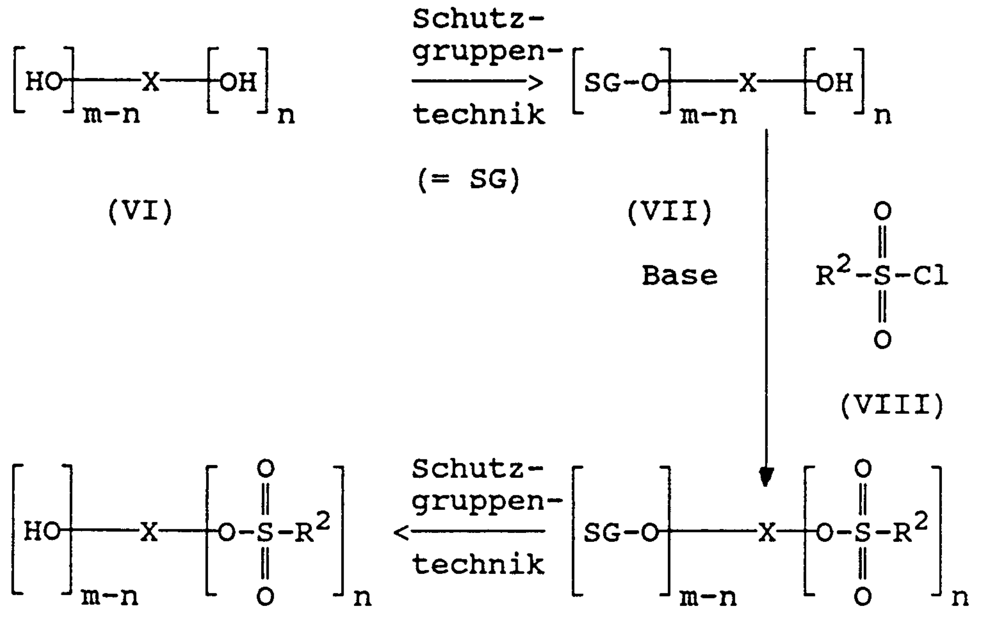


(VI) (VII) (VIII)

(IV) (IX)

2) Entsprechend dem Schema 5 erfolgt die Umsetzung eines mehrwertigen Alkohols der allgemeinen Formel VI mit der gewünschten Menge eines monofunktionellen Sulfonsäurechlorids der allgemeinen Formel VIII zu mono- (m = 3, 4, 5), bis- (m = 6, 8) und trifunktionellen (m = 8) Sulfonsäureestern und/oder einem Isomerengemisch verschiedener sulfonsäureesterhaltiger Alkohole der allgemeinen Formel IV. Die Herstellung von Sulfonsäureestern (Sulfonaten) aus Alkoholen ist für monofunktionelle Verbindungen bekannt und wird beispielsweise von E. Schaumann in "Methoden der organischen Chemie" (Houben-Weyl-Müller, Eds.), 4. Aufl., Bd. 6/1b, S. 874, Thieme-Verlag, Stuttgart 1984, und dort zitierte Literatur) ausführlich beschrieben.

Schema 5

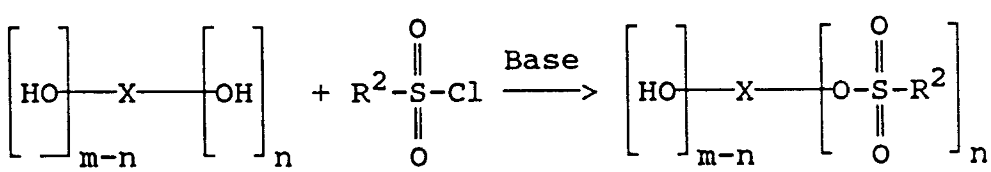


(VI) (VIII) (IV)

Bei der Reaktionsfolge nach Schema 4 wird der betreffende, mehrfunktionelle Alkohol der allgemeinen Formel VI mit der zum gewünschten Umsetzungsgrad erforderlichen Menge eines geeigneten Schutzgruppenreagenzes und einer katalytischen Menge einer Säure versetzt und man erhält partiell geschützte Alkohole der allgemeinen Formel VII. Art und Menge der verwendeten Schutzgruppenreagenzien richten sich nach der Struktur der mehrfunktionellen Alkohole VI. Die Säure und die gewählten Reaktionsbedingungen richten sich nach dem verwendeten Schutzgruppenreagenz. Da bei der Bildung der geschützten Sulfonsäureester der allgemeinen Formel IX Basen zur Deprotonierung eingesetzt werden, müssen die geschützten Alkohole der allgemeinen Formel VII basenstabil sein. Umsetzungsprodukte von Alkoholen mit schutzgruppenbildenden Reagenzien sind vor allem substituierte Methyl- und Ethylether, Silylether, Ester, Carbonate, cyclische Acetale und Ketale sowie cyclische Orthoester. Für

Alkohole der allgemeinen Formel VI, die 1,2- oder 1,3-Hydroxylgruppen enthalten, kommen cyclische Acetale und Ketale sowie Orthoester in Frage. Bevorzugt sind Reagenzien, die zur Bildung cyclischer Ketale führen, wobei acetonid- (isopropyliden)geschützte 1,2- und 1,3-Alkohole besonders bevorzugt sind.

Acetonide (1,2- bzw. 1,3-O,O-Isopropylidenketale) können durch Umsetzung von 1,2- und 1,3-Alkoholen der allgemeinen Formel VI mit z. B. 2-Methoxypropen, 2-Ethoxypropen, 2-(Trimethylsilyloxy)propen, Aceton oder 2,2-Dimethoxypropan in Gegenwart katalytischer Mengen an Säure erhalten werden. Besonders bevorzugt ist die Umsetzung von Alkoholen, enthaltend 1,2- bzw. 1,3-Hydroxygruppen, in wasserfreiem Aceton, da hier das schutzgruppenbildende Reagenz gleichzeitig als Lösungsmittel fungiert.

Bei der Reaktionsfolge nach Schema 4 werden die verwendeten Alkohole, die 1,2- oder 1,3-Hydroxylgruppen enthalten, mit einer 2- bis 20fachen, bevorzugt 5-bis 12fachen, Menge an Aceton erwärmt. Gegebenenfalls können noch ein oder auch mehrere weitere Lösungsmittel zugesetzt werden, um eine bessere Löslichkeit zu erzielen oder die Reaktionsgeschwindigkeit entsprechend zu erhöhen. Nach Zugabe einer katalytischen Menge an Säure, bevorzugt p-Toluolsulfonsäure, wird die Mischung 1 bis 24 h, bevorzugt 3 bis 8 h bei 20 bis 85 °C, bevorzugt bei 35 bis 60 °C, gerührt und anschließend mit der 1- bis 10fachen, bevorzugt 2-bis 5fachen, Menge an Kaliumcarbonat (bezogen auf die eigesetzte Menge an p-Toluolsulfonsäure) oder einer anderen Base versetzt.

Nach dem Abkühlen können die meist trüben Mischungen über ein Trennhilfsmittel, wie Kieselgur, Kieselgel o. a., filtriert werden. Anschließend destilliert man das Lösungsmittel im Vakuum ab. Das Produkt wird nach den durch den Fachmann bekannten Methoden, z. B. durch Destillation im Hochvakuum, gereinigt.

Mehrfunktionelle Alkohole der allgemeinen Formel VI, die keine 1,2- bzw. 1,3-Hydroxygruppen aufweisen, werden besonders durch die Umwandlung in Ether geschützt. Dabei sind substituierte Methylether und Silylether bevorzugt. Besonders bevorzugt sind die Tetrahydropyranylether. Diese werden bevorzugt auf zwei verschiedene Weisen synthetisiert, die von A. Bongini, G. Cardillo, M. Orena, S. Sandri, Synthesis **1979**, 618 (Var. A) bzw. von M. Miyashita, A. Yoshikoshi, P. A. Grieco, J. Org. Chem. **42** (1977) 3772 (Var. B) beschrieben wurden.

Variante A:

Bei der Reaktionsfolge nach Schema 4 werden die verwendeten Alkohole, die keine 1,2- oder 1,3-Hydroxylgruppen enthalten, mit einer dem gewünschten Umsetzungsgrad entsprechenden Menge an 3,4-Dihydro-2H-pyran in einer 1- bis 20fachen, bevorzugt 2- bis 10fachen, Menge Hexan gelöst und zu einer Suspension aus saurem Ionenaustauschharz (Amberlyst H-15, 0,05- bis 2fache, bevorzugt 0,1- bis 0,5fache Menge, bezogen auf die eingesetzte Menge an Hydroxylgruppen) in Hexan (Menge wie oben) gegeben. Gegebenenfalls können noch ein oder auch mehrere weitere Lösungsmittel, wie z. B. Methanol, zugesetzt werden, um eine bessere Löslichkeit zu erzielen. Die Mischung wird 0,5 bis 24 h, bevorzugt 1 bis 5 h bei 0 bis 85 °C, bevorzugt bei 20 bis 50 °C, gerührt und anschließend das Harz abfiltriert. Anschließend destilliert man das Lösungsmittel im Vakuum ab. Der verbleibende Rückstand, der praktisch ausschließlich die isomeren Alkohole enthält, wird durch Chromatographie aufgetrennt.

Variante B:

Bei der Reaktionsfolge nach Schema 4 werden die verwendeten Alkohole, die keine 1,2- oder 1,3-Hydroxylgruppen enthalten, mit einer dem gewünschten Umsetzungsgrad entsprechenden Menge an 3,4-Dihydro-2H-pyran in einer 5- bis 300fachen, bevorzugt 20- bis 100fachen, Menge Dichlormethan gelöst und mit Pyridinium-p-Toluolsulfonat (0,02- bis 1,0fache, bevorzugt 0,05- bis 0,3fache, Molmenge, bezogen auf die Anzahl der umzusetzenden Hydroxylgruppen) versetzt. Gegebenenfalls können noch weitere Lösungsmittel zugesetzt werden. Anschließend wird die Mischung 0,5 bis 48 h, bevorzugt 2,5 bis 8 h, bei 0 bis 80 °C, bevorzugt bei 10 bis 45 °C, gerührt und anschließend mit der 5- bis 100fachen, bevorzugt 10- bis 50fachen, Menge Ether (bezogen auf die eingesetzte Lösungsmittelmenge) versetzt. Man wäscht mit partiell gesättigten Lösungen von Natriumchlorid und mit Wasser. Nach dem Trocknen mit Natriumsulfat wird das Lösungsmittel im Vakuum abdestilliert. Der verbleibende Rückstand, der praktisch ausschließlich die isomeren Alkohole enthält, wird durch Chromatographie aufgetrennt.

Der dem gewünschten Umsetzungsgrad entsprechende, partiell geschützte Alkohol der allgemeinen Formel VII wird nach beiden Varianten als Hauptprodukt erhalten.

Die noch freie(n) Hydroxylgruppe(n) der partiell geschützten Alkohole der allgemeinen Formel VII werden mit Sulfonsäurechloriden (Sulfonylchloriden) der allgemeinen Formel VIII unter Einfluß von Base

umgesetzt. Man erhält die geschützten Alkohol-Sulfonsäureester der allgemeinen Formel IX.

Bei der Reaktionsfolge nach Schema 4 werden die partiell geschützten Alkohole der allgemeinen Formel VII mit einer 0,5- bis 40fachen, bevorzugt 2- bis 10fachen, Menge einer Base gelöst und 0,1 bis 10 h, bevorzugt 0,5 bis 1 h bei -40 bis 65 °C, bevorzugt bei -15 bis 25 °C, gerührt. Als Basen werden vor allem basische Amine, wie z. B. Pyridin, Triethylamin, N-Methylmorpholin u. a. bevorzugt, ganz besonders bevorzugt ist jedoch N-Methylmorpholin. Gegebenenfalls können noch ein oder auch mehrere weitere Lösungsmittel, wie z. B. Acetonitril, zugesetzt werden, um z. B. eine bessere Löslichkeit oder eine Erhöhung der Reaktionsgeschwindigkeit zu erzielen. Anschließend versetzt man die Mischung mit dem gewünschten Sulfonsäurechlorid VIII (1- bis 3fache, bevorzugt 1,05- bis 1,5fache, Menge, bezogen auf die im eingesetzten Alkohol noch vorhandenen Hydroxylgruppen) und rührt 0,5 bis 48 h, bevorzugt 2 bis 8 h bei -40 bis 65 °C, bevorzugt bei -15 bis 25 °C. Anschließend versetzt man die Mischung mit Dichlormethan/Wasser, wäscht die organische Phase, nach den durch den Fachmann bekannten Methoden, neutral, trocknet mit Natriumsulfat und destilliert das Lösungsmittel im Vakuum ab.

Obwohl die verbleibenden Rückstände hauptsächlich aus den partiell geschützten Alkohol-Sulfonsäureestern der allgemeinen Formel IX bestehen - und in der Regel ohne weitere Reinigung in der nächsten Stufe eingesetzt werden - kann das Produkt ggf. weiterhin nach den durch den Fachmann bekannten Methoden gereinigt werden.

Bei der Reaktionsfolge nach Schema 4 werden die Schutzgruppen der partiell geschützten Alkohol-Sulfonsäureester der allgemeinen Formel IX in Anwesenheit einer katalytischen Menge Säure vollständig entfernt. Man erhält die Alkohol-Sulfonsäureester der allgemeinen Formel IV. Art und Menge der verwendeten Säure richten sich nach der oben eingesetzten Schutzgruppe. So werden Acetonid-Schutzgruppen (O,O-Isopropylidenacetale) bevorzugt mit wäßrigen HCl-Lösungen unterschiedlicher Konzentration, mit Essigsäure, mit p-Toluolsulfonsäure und/oder durch den Einsatz saurer Ionenaustauschharze entfernt. Für die Spaltung der Tetrahydropyranylether in die entsprechenden Alkohole finden die gleichen Reagenzien Anwendung.

Bei der Spaltung der cyclischen Ether gemäß Schema 4 werden die partiell geschützten Alkohol-Sulfonsäureester der allgemeinen Formel IX in einer 5- bis 200fachen, bevorzugt 10- bis 50fachen, Menge eines Lösungsmittels gelöst und so lange mit wäßriger Salzsäure versetzt, bis der pH $\approx$ 1 ist. Als Lösungsmittel finden Alkohole, Ether, Wasser, organische Säuren, Ketone oder Mischungen dieser Solventien Verwendung. Bevorzugt sind Alkohole und Ether, wie z. B. Methanol oder Tetrahydrofuran, aber auch Mischungen, bestehend aus Tetrahydrofuran, Essigsäure oder verdünnter Salzsäure und Wasser. Besonders bevorzugt ist Methanol. Anschließend versetzt man mit einer katalytischen Menge p-Toluolsulfonsäure und rührt 1 bis 18 h, bevorzugt 2 bis 7 h bei -25 bis 80 °C, bevorzugt bei 0 bis 45 °C, gerührt. Anschließend stellt man durch Zugabe alkoholischer Kaliumhydroxid-Lösung den pH-Wert der Mischung auf schwach alkalisch ein (pH $\approx$ 8 bis 9) und destilliert das Lösungsmittel im Vakuum ab. Der Rückstand wird durch Säulenchromatographie an Kieselgel aufgetrennt, und man erhält die gewünschten Alkohol-Sulfonsäureester der allgemeinen Formel IV meist als farblose Verbindungen. Als Laufmittel für die Säulenchromatographie finden bevorzugt Dichlormethan, Essigester, Ether, Hexan, Petrolether verschiedener Siedefraktionen, Tetrahydrofuran, Trichlormethan und Gemische dieser Eluenten Verwendung. Besonders bevorzugt sind Dichlormethan und dessen Mischungen.

Entsprechend dem Schema 5 kann die Umsetzung eines mehrwertigen Alkohols der allgemeinen Formel VI, der keine 1,2- oder 1,3-Hydroxylgruppen enthält, mit Sulfonsäurechloriden auch ohne Schutzgruppentechnologie erfolgen. Dabei ergibt die Reaktion mit der gewünschten Menge eines monofunktionellen Sulfonsäurechlorids der allgemeinen Formel VIII mono-(m = 3, 4, 5), bis- (m = 6, 8) und trifunktionelle (m = 8) Sulfonsäureester und/oder ein Isomerengemisch verschiedener sulfonsäureesterhaltiger Alkohole der allgemeinen Formel IV.

Für diese Umsetzungen gelten die gleichen Bedingungen, wie sie für den Reaktionsschritt VII → IX gemäß Schema 4 explizit erläutert wurden.

Als Alkohole der allgemeinen Formel VI können tri-, tetra- oder höherfunktionelle Alkohole Verwendung finden; bevorzugt sind Alkohole, die 3 bis 6 OH-Gruppen pro Molekül enthalten.

Trifunktionelle Alkohole leiten sich bevorzugt vom Glycerin, von höheren $\alpha,\beta,\omega$-Triolen oder vom Triethanolamin ab, wobei auch hier höherkettige Derivate, insbesondere ethoxylierte Verbindungen, wie z. B. 1,3,5-Tris(2-hydroxyethyl)cyanursäure gleichermaßen Verwendung finden können. Genannt werden Glycerin, 2-Hydroxymethyl-2-methyl-propan-1,3-diol, 2-Ethyl-2-hydroxymethyl-propan-1,3-diol, 2,3-Isopropyliden-erythruronsäure, Hexan-1,2,6-triol, 1,1,1-Triethanolamin, 1,1,1-Tripropanolamin sowie partiell acetalisierte oder ketalisierte Zuckerderivate.

Darüber hinaus lassen sich auch Umsetzungsprodukte aus tetrafunktionellen Alkoholen oder Aminotriolen mit Säurederivaten, Isocyanaten oder cyclischen Carbonaten verwenden. Höherwertige Alkohole der

allgemeinen Formel VI leiten sich beispielsweise von Kondensationsprodukten des Glycerins, des Pentaerythrits oder von Umsetzungsprodukten bifunktioneller Säurederivate oder Isocyanate mit höherwertigen Alkoholen oder Aminoalkoholen ab.

Die Liste der verwendbaren Alkohole ist damit durchaus nicht vollständig; es lassen sich praktisch alle Alkohole verwenden, die keine weitere mit Säureestern reagierende Gruppe aufweisen oder die unter den beschriebenen Umsetzungsbedingungen eindeutig unter Esterbildung reagieren.

Bei der Herstellung von β-Ketoester-Sulfonsäureestern der allgemeinen Formel II durch Umesterung gemaß Schema 3 werden monofunktionelle β-Ketoester der allgemeinen Formel V mit Alkohol-Sulfonsäureestern der allgemeinen Formel IV in der Weise eingesetzt, daß ein 5 bis 200%iger, bevorzugt ein 10 bis 50%iger, Überschuß eines mit einem niedermolekularen Alkohol veresterten β-Ketoesters, z.B. eines Methyl- oder Ethylesters, bei 100 bis 160 °C, bevorzugt bei 120 bis 140 °C, umgesetzt wird. Zur Erhöhung der Löslichkeit des Alkohol-Sulfonsäureesters der allgemeinen Formel IV im β-Ketoester der allgemeinen Formel V kann ggf. ein Lösungsvermittler, wie Dimethylformamid oder N-Methylpyrrolidon, zugesetzt werden. Durch Anlegen eines schwachen Vakuums von 800 bis 20 Torr, bevorzugt von 400 bis 100 Torr, wird das Gleichgewicht durch Abdestillieren des gebildeten niederen Alkohols kontinuierlich in die gewünschte Richtung verschoben. Nachdem die theoretische Menge an niederem Alkohol abdestilliert ist, wird der überschüssige, niedrig veresterte β-Ketoester der allgemeinen Formel V sowie ggf. der zugefügte Lösungsvermittler im Hochvakuum abdestilliert. Als Rückstand verbleibt der häufig ölig anfallende β-Ketoester-Sulfonsäureester der allgemeinen Formel II in zumeist sehr hoher Reinheit, so daß er ohne weitere Reinigung für den Diazotransfer verwendet werden kann.

Die bei dieser Reaktionsfolge erforderlichen niedrig veresterten β-Ketoester der allgemeinen Formel V sind z. T. kommerziell verfügbar oder lassen sich nach zahlreichen literaturbekannten Methoden herstellen. Besonders bevorzugt ist dabei ihre Herstellung aus den entsprechenden Derivaten der 5-Acyl-meldrumsäure der allgemeinen Formel III. Obwohl hierbei ein zusätzlicher Reaktionsschritt gegenüber der Verfahrensvariante 1 beschritten wird, lassen sich mit dieser Variante verbesserte Ausbeuten und/oder reinere β-Ketoester-Sulfonsäureester der allgemeinen Formel II erzielen.

Die genannten erfindungsgemäßen Verbindungen werden in strahlungsempfindlichen Gemischen verwendet. Sie weisen eine hohe Lichtempfindlichkeit auf, insbesondere mit Licht einer Wellenlänge zwischen 190 und 300 nm. Da die Gemische bei Belichtung sehr gut ausbleichen, lassen sich Strukturierungen erzielen, die denen der bekannten Gemische hinsichtlich ihres Auflösevermögens deutlich überlegen sind. Die Anwendung der erfindungsgemäßen strahlungsempfindlichen Verbindungen wird in der gleichzeitig eingereichten deutschen Patentanmeldung P 40 14 648.0 beschrieben.

Beispiel 1

Herstellung eines trifunktionellen Esters mit zwei α-Diazo-β-ketoester-Einheiten und einer Sulfonsäureester-Einheit der allgemeinen Formel I: 4-tert-Butylphenylsulfonsäureester des 2,10-Diazo-1,11-dicyclohexyl-4,8-dioxa-6-ethyl-6-(hydroxymethyl)-1,3,9,11-tetraoxoundecans (**5**)

Stufe 1:

2,2-Dimethyl-5-ethyl-5-(hydroxymethyl)-1,3-dioxan (**1**)

Zu 275 g (2,05 mol) 2,2-Bis(hydroxymethyl)-1-butanol in 1,4 l Aceton gibt man 2,75 g p-Toluolsulfonsäure und erhitzt zum Rückfluß. Nach 4 h neutralisiert man die noch heiße Lösung durch Zugabe von 8,25 g Kaliumcarbonat und rührt weitere 30 min; anschließend läßt man über Nacht bei Raumtemperatur stehen. Die trübe Mischung wird über Kieselgur filtriert und das Lösungsmittel im Vakuum abdestilliert. Es verbleiben 400 ml eines trüben, öligen Rückstands, der durch Destillation im Hochvakuum gereinigt wird. Man erhält 224 g des Alkohols **1** als farbloses Öl.

Stufe 2:

4-tert-Butylphenylsulfonsäureester des 2,2-Dimethyl-5-ethyl-5-(hydroxymethyl)-1,3-dioxans (**2**)

6,97 g (40 mmol) der vorstehend beschriebenen Verbindung **1** werden mit 8,6 g (85 mmol) N-Methylmorpholin in 50 ml Acetonitril eingetragen, auf 0 °C gekühlt und gerührt. Nach 30 min werden 10,0 g (43 mmol) 4-tert-Butylphenylsulfonsäurechlorid zur Lösung gegeben. Man rührt 7 h bei 0 °C und läßt die Mischung über Nacht bei -20 °C stehen. Nach Erwärmen auf Raumtemperatur wird das Lösungsmittel im

Vakuum abdestilliert. Der verbleibende, ölige Rückstand wird mit 150 ml Wasser und mit 100 ml Dichlormethan versetzt. Die organische Phase wird abgetrennt, die wäßrige Phase mit Dichlormethan nachextrahiert. Die vereinigten organischen Phasen werden dreimal mit je 100 ml halbgesättigter Kaliumdihydrogenphosphat-Lösung und zweimal mit je 150 ml Wasser gewaschen, über Natriumsulfat getrocknet und das Lösungsmittel im Vakuum abdestilliert. Es verbleiben 12 g eines gelben Öls, das praktisch ausschließlich aus dem geschützten Sulfonsäureester **2** besteht. Er wird ohne weitere Reinigung als Ausgangsprodukt für die nächste Stufe verwendet.

Stufe 3:

4-tert-Butylphenylsulfonsäureester des 2,2-Bis-(hydroxymethyl)-1-butanols (**3**)

11,1 g (30 mmol) des in Stufe 2 hergestellten Sulfonsäureesters **2** werden in 100 ml Methanol eingetragen und es wird so lange 3 N Salzsäure zugetropft, bis der pH-Wert kleiner als fünf ist. Anschließend gibt man 250 mg p-Toluolsulfonsäure zu, rührt 4,5 h bei Raumtemperatur und versetzt die Mischung so lange mit methanolischer Kaliumhydroxid-Lösung bis die Mischung schwach alkalisch (pH ≈ 8 bis 9) reagiert. Zu einer Zwischenverbindung 4 werden dann unter Rühren 2,37 g (12 mmol) Tosylazid hinzugefügt und anschließend 2,5 g (25 mmol) Triethylamin derart zugetropft, daß die Temperatur nicht über 5 °C ansteigt. Die Mischung wird 2 h bei dieser Temperatur gerührt; anschließend wird auf Raumtemperatur erwärmt. Nach 2 h ist in der Mischung dünnschichtchromatographisch (Kieselgel, Laufmittel: Dichlormethan) kein Tosylazid mehr nachweisbar. Die Mischung wird bei O °C mit 1,70 g (7,5 mmol) 4-Carboxyphenylsulfonylazid versetzt und 15 min gerührt. Nach weiteren 2 h bei Raumtemperatur wird das Lösungsmittel im Vakuum abdestilliert. Der Rückstand wird in 100 ml Dichlormethan aufgenommen und viermal mit je 50 ml einer 25%igen wäßrigen Kaliumhydroxid-Lösung gewaschen. Anschließend wäscht man mit Wasser neutral und trocknet die organische Phase über Natriumsulfat. Nach dem Einengen verbleibt ein orangerotes Öl, das durch Anlegen eines Hochvakuums von Lösungsmittelresten weitgehend befreit wird. Nach einer Säulenchromatographie an Kieselgel mit Dichlormethan/Hexan (1:1) als Laufmittel erhält man 1,8 g nahezu farbloses Produkt **5**, welches folgende Zusammensetzung aufweist.

| | | | | | |
|---|---|---|---|---|---|
| $C_{34}H_{46}N_4O_9S$ (MG 686,8) | Ber.:<br>Gef.: | C 59,5 %<br>C 59,9 % | H 6,8 %<br>H 6,8 % | N 8,2 %<br>N 8,0 % | S 4,7 %<br>S 4,4 % |

IR (KBr): 2.136 $cm^{-1}$ ($C=N_2$)

Beispiel 2

Herstellung eines trifunktionellen Esters mit zwei α-Diazo-β-ketoester-Einheiten und einer Sulfonsäureester-Einheit der allgemeinen Formel I: 2-(Benzoylaminomethyl)thiophen-5-sulfonsäureester des 2,10-Diazo-1,11-dicyclohexyl-4,8-dioxa-6-ethyl-6-(hydroxymethyl)-1,3,9,11-tetraoxoundecans (**9**)

Stufe 1:

2,2-Dimethyl-5-ethyl-5-(hydroxymethyl)-1,3-dioxan (**1**) Herstellung siehe Beispiel 1, Stufe 1;

Stufe 2:

2-(Benzoylaminomethyl)thiophen-5-sulfonsäureester des 2,2-Dimethyl-5-ethyl-5-(hydroxymethyl)-1,3-dioxans (**6**)

3,26 g (18,7 mmol) des partiell geschützten Alkohols **1** und 8,09 g (80 mmol) N-Methylmorpholin werden auf 0 °C gekühlt und gerührt. Nach 10 min werden 6,5 g (21 mmol) 2-(Benzoylaminomethyl)-thiophen-5-sulfonsäurechlorid portionsweise zugegeben. Man rührt 3 h bei 0 °C, 30 min bei Raumtemperatur und versetzt mit 30 ml Dichlormethan/150 ml Eiswasser. Die organische Phase wird abgetrennt und die wäßrige Phase dreimal mit je 50 ml Dichlormethan extrahiert. Die vereinigten organischen Phasen werden neutral gewaschen, mit Natriumsulfat getrocknet und das Lösungsmittel im Vakuum abdestilliert. Im Hochvakuum werden die letzten Lösungsmittelreste nahezu vollständig entfernt und es verbleiben 7,5 g eines tiefroten, zähen Öls, das den Sulfonsäureester **6** enthält und ohne weitere Reinigung als Ausgangsprodukt für die nächste Stufe verwendet wird.

Stufe 3:

2-(Benzoylaminomethyl)thiophen-5-sulfonsäureester des 2,2-Bis(hydroxymethyl)-1-butanols (**7**)

7,8 g (17 mmol) des in Stufe 2 hergestellten, rohen Sulfonsäureesters **6** werden in 60 ml Methanol eingetragen und es wird so lange 3 N Salzsäure zugetropft, bis der pH-Wert kleiner als fünf ist. Anschließend gibt man 400 mg p-Toluolsulfonsäure zu, rührt 21 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert und der verbleibende Rückstand wird durch Säulenchromatographie an Kieselgel mit Dichlormethan als Laufmittel aufgetrennt. Man erhält 4,1 g farbloses Öl **7**.

Stufe 4:

2-(Benzoylaminomethyl)thiophen-5-sulfonsäureester des 1,11-Dicyclohexyl-4,8-dioxa-6-ethyl-6-(hydroxymethyl)-1,3,9,11-tetraoxoundecans (**8**)

4,1 g (9,9 mmol) der vorstehend beschriebenen Verbindung **7** werden mit 5,3 g (20,8 mmol) 5-(1-Cyclohexyl-1-hydroxymethyliden)-2,2-dimethyl-1,3-di oxan-4,6-dion in 20 ml Aceton eingetragen und langsam erwärmt. Ab etwa 60 °C beginnt eine starke Kohlendioxid-Entwicklung. Die Lösung wird für 2 h am Rückfluß gehalten und anschließend das Lösungsmittel im Vakuum abdestilliert. Nach Abkühlung verbleibt ein öliger, schwarzbrauner Rückstand, der - neben anderen Verbindungen - als Hauptprodukt den gewünschten β-Ketoester-Sulfonsäureester **8** enthält. Er wird ohne weitere Reinigung als Ausgangsprodukt für die nächste Stufe verwendet.

Stufe 5:

2-(Benzoylaminomethyl)thiophen-5-sulfonsäureeSter des 2,10-Diazo-1,11-dicyclohexyl-4,8-dioxa-6-ethyl-6-(hydroxymethyl)-1,3,9,11-tetraoxoundecans (**9**)

Die vorstehend beschriebene Verbindung **8** wird in der aus Stufe 4 anfallenden Menge in 35 ml Acetonitril gelöst und auf 0 °C gekühlt. Zu der erkalteten Lösung werden unter Rühren 2,76 g (14 mmol) Tosylazid hinzugefügt und anschließend 2,02 g (20 mmol) Triethylamin derart zugetropft, daß die Temperatur nicht über 5 °C ansteigt. Die Mischung wird 1 h bei dieser Temperatur gerührt; anschließend wird auf Raumtemperatur erwärmt. Nach 2 h ist in der Mischung dünnschichtchromatographisch (Kieselgel, Laufmittel: Dichlormethan) kein Tosylazid mehr nachweisbar. Die Mischung wird bei O °C mit 1,36 g (6,0 mmol) 4-Carboxyphenylsulfonylazid versetzt und 30 min gerührt. Nach 1 h bei Raumtemperatur wird die Mischung über Nacht bei -20 °C eingefroren. Nach Erwärmen auf Raumtemperatur wird das Lösungsmittel im Vakuum abdestilliert, der Rückstand in 120 ml Dichlormethan aufgenommen und viermal mit je 40 ml 20%iger wäßriger Kaliumhydroxid-Lösung gewaschen. Anschließend wäscht man mit Wasser neutral und trocknet die organische Phase mit Natriumsulfat. Nach dem Einengen verbleibt ein tiefrotes Öl, das durch Anlegen eines Hochvakuums von Lösungsmittelresten weitgehend befreit wird. Nach einer Säulenchromatographie an Kieselgel mit Dichlormethan als Laufmittel erhält man 1,6 g des α-Diazo-β-ketoester-Sulfonsäureesters **9**, welcher folgende Zusammensetzung aufweist.

| | | | | | |
|---|---|---|---|---|---|
| $C_{36}H_{43}N_5O_{10}S_2$ (MG 769,9) | Ber.:<br>Gef.: | C 56,2 %<br>C 55,7 % | H 5,6 %<br>H 5,8 % | N 9,1 %<br>N 8,3 % | S 8,3 %<br>S 8,2 % |

IR (KBr): 2.137 $cm^{-1}$ ($C=N_2$)

<u>Beispiel 3</u>

Herstellung eines trifunktionellen Esters mit zwei α-Diazo-β-ketoester-Einheiten und einer Sulfonsäureester-Einheit der allgemeinen Formel I: 4-Bromphenylsulfonsäureester des 4,12-Diazo-6,10-dioxa-1,15-diphenyl-8-ethyl-8-(hydroxymethyl)-3,5,11,13-tetraoxopentadecans (**13**)

Stufe 1:

2,2-Dimethyl-5-ethyl-5-(hydroxymethyl)-1,3-dioxan (**1**) Herstellung siehe Beispiel 1, Stufe 1;

Stufe 2:

4-Bromphenylsulfonsäureester des 2,2-Dimethyl-5-ethyl-5-(hydroxymethyl)-1,3-dioxans (**10**)

31,4 g (0,18 mol) des partiell geschützten Alkohols **1** werden in 81 g (0,80 mol) N-Methylmorpholin eingetragen, auf 0 °C gekühlt und gerührt. Nach 30 min werden 51 g (0,20 mol) 4-Bromphenylsulfonsäurechlorid zur Lösung gegeben. Man rührt 4 h bei 0 °C und bewahrt die Mischung über Nacht bei -20 °C auf. Nach Erwärmen auf Raumtemperatur versetzt man mit 1,0 l Eiswasser/500 ml Dichlormethan. Die organische Phase wird abgetrennt, die wäßrige Phase mit Dichlormethan nachextrahiert. Die vereinigten organischen Phasen werden viermal mit je 250 ml Wasser gewaschen, mit Natriumsulfat getrocknet und das Lösungsmittel im Vakuum abdestilliert. Es verbleiben 74 g eines gelben Öls **10**, das über Nacht kristallisiert. Der geschützte Sulfonsäureester **10** wird ohne weitere Reinigung als Ausgangsprodukt für die nächste Stufe verwendet.

Stufe 3:

4-Bromphenylsulfonsäureester des 2,2-Bis(hydroxymethyl)-1-butanols (**11**)

39,5 g (0,10 mol) des in Stufe 2 hergestellten, geschützten Sulfonsäureesters **10** werden in 600 ml Methanol vollständig gelöst und es wird so lange 3 N Salzsäure zugetropft, bis der pH-Wert kleiner als fünf ist. Anschließend gibt man 4,0 g p-Toluolsulfonsäure zu, rührt über Nacht bei Raumtemperatur und versetzt die Mischung so lange mit methanolischer Kaliumhydroxid-Lösung bis sie schwach alkalisch (pH ≈ 8 bis 9) reagiert. Das Lösungsmittel wird im Vakuum abdestilliert und der verbleibende Rückstand durch Säulenchromatographie an Kieselgel mit Dichlormethan als Laufmittel aufgetrennt. Man erhält 10,2 g kristallinen Sulfonsäureester **11**.

Stufe 4:

4-Bromphenylsulfonsäureester des 6,10-Dioxa-1,15-diphenyl-8-ethyl-8-(hydroxymethyl)-3,5,11,13-tetraoxopentadecans (**12**)

10,0 g (28,3 mmol) der vorstehend beschriebenen Verbindung **11** werden mit 15,6 g (56,5 mmol) 5-(3-Phenyl-1-hydroxypropyliden)-2,2-dimethyl-1,3-dioxan-4,6-dion in 100 ml Aceton eingetragen und langsam erwärmt. Ab etwa 60 °C beginnt eine starke Kohlendioxid-Entwicklung. Die Lösung wird für 2 h am Rückfluß gehalten und anschließend das Lösungsmittel im Vakuum abdestilliert. Nach Abkühlung der Reaktionsmischung verbleibt ein zähes, tiefrotes Öl, das den gewünschten β-Ketoester-Sulfonsäureester **12** als Hauptprodukt enthält. Der β-Ketoester-Sulfonsäureester **12** wird ohne weitere Reinigung als Ausgangsprodukt für die nächste Stufe verwendet.

Stufe 5:

4-Bromphenylsulfonsäureester des 4,12-Diazo-6,10-dioxa-1,15-diphenyl-8-ethyl-8-(hydroxymethyl)-3,5,11,13-tetraoxopentadecans (**13**)

Die vorstehend beschriebene Verbindung **12** wird in der in Stufe 4 anfallenden Menge in 250 ml Acetonitril gelöst und auf 0 °C gekühlt. Zu der erkalteten Lösung werden unter Rühren 9,38 g (47,6 mmol) Tosylazid hinzugefügt und anschließend 6,76 g (67 mmol) Triethylamin derart zugetropft, daß die Temperatur nicht über 5 °C ansteigt. Die Mischung wird 1 h bei dieser Temperatur gerührt; anschließend wird auf Raumtemperatur erwärmt und weitere 2 h bei dieser Temperatur gerührt. Die Mischung wird bei 0 °C mit 4,33 g (19 mmol) 4-Carboxyphenylsulfonylazid versetzt und 15 min gerührt. Nach weiteren 2 h bei Raumtemperatur wird das Lösungsmittel im Vakuum abdestilliert. Der Rückstand wird in Ether aufgenommen und dreimal mit je 200 ml wäßriger Kaliumhydroxid-Lösung (insgesamt 12 g Kaliumhydroxid in 600 ml Wasser) gewaschen. Anschließend wäscht man mit Wasser neutral und trocknet die organische Phase mit Natriumsulfat. Nach dem Einengen verbleibt ein zähviskoses Öl, das durch Anlegen eines Hochvakuums

von Lösungsmittelresten weitgehend befreit wird. Nach einer Säulenchromatographie an Kieselgel mit Dichlormethan als Laufmittel erhält man den schwach gelblichen α-Diazo-β-ketoester-Sulfonsäureester **13**, der folgende Zusammensetzung aufweist.

| | | | | | |
|---|---|---|---|---|---|
| $C_{34}H_{33}BrN_4O_9S$ (MG 753,6) | Ber.:<br>Gef.: | C 54,2 %<br>C 54,6 % | H 4,4 %<br>H 4,2 % | N 7,4 %<br>N 7,1 % | S 4,3 %<br>S 4,4 % |

IR (KBr): 2.140 $cm^{-1}$ ($C=N_2$)

Beispiel 4

Herstellung eines trifunktionellen Esters mit zwei α-Diazo-β-ketoester-Einheiten und einer Sulfonsäureester-Einheit der allgemeinen Formel I: 3-Chlorpropylsulfonsäureester des 1,13-Bis(phenoxy)-3,11-diazo-5,9-dioxa-7-ethyl-7-(hydroxymethyl)-2,4,10,12-tetraoxotridecans (**17**)

Stufe 1:

2,2-Dimethyl-5-ethyl-5-(hydroxymethyl)-1,3-dioxan (**1**)

Herstellung siehe Beispiel 1, Stufe 1;

Stufe 2:

3-Chlorpropylsulfonsäureester des 2,2-Dimethyl-5-ethyl-5-(hydroxymethyl)-1,3-dioxans (**14**)

8,71 g (50 mmol) des partiell geschützten Alkohols **1** werden in 11,1 g (0,11 mol) N-Methylmorpholin eingetragen, auf 0 °C gekühlt und gerührt. Nach 30 min werden 9,74 g (55 mmol) 3-Chlorpropylsulfonsäurechlorid zur Lösung gegeben. Man rührt 8 h bei 0 °C und bewahrt die Mischung über Nacht bei -20 °C auf. Nach Erwärmen auf Raumtemperatur wird das Lösungsmittel im Vakuum abdestilliert. Der verbleibende, ölige Rückstand wird mit 250 ml Wasser und mit 200 ml Dichlormethan versetzt. Die organische Phase wird abgetrennt, die wäßrige Phase mit Dichlormethan nachextrahiert. Die vereinigten organischen Phasen werden dreimal mit je 100 ml halbgesättigter Kaliumdihydrogenphosphat-Lösung und zweimal mit je 150 ml Wasser gewaschen, mit Natriumsulfat getrocknet und das Lösungsmittel im Vakuum abdestilliert. Es verbleiben 13 g eines gelben Öls, das den gewünschten Sulfonsäureester **14** enthält und ohne weitere Reinigung als Ausgangsprodukt für die nächste Stufe verwendet wird.

Stufe 3:

3-Chlorpropylsulfonsäureester des 2,2-Bis(hydroxymethyl)-1-butanols (**15**)

11,1 g (40 mmol) des in Stufe 2 hergestellten geschützten Sulfonsäureesters **14** werden in 150 ml Methanol/50 ml Tetrahydrofuran eingetragen, und es wird so lange 3 N Salzsäure zugetropft, bis der pH-Wert kleiner als fünf ist. Anschließend gibt man 0,50 g p-Toluolsulfonsäure zu, rührt 3,5 h bei Raumtemperatur und versetzt die Mischung so lange mit methanolischer Kaliumhydroxid-Lösung bis sie schwach alkalisch (pH ≈ 8 bis 9) reagiert. Das Lösungsmittel wird im Vakuum abdestilliert. Es verbleibt ein gelboranger, öliger Rückstand, der durch Säulenchromatographie an Kieselgel mit Dichlormethan als Laufmittel aufgetrennt wird. Man erhält 7,2 g farbloses Öl **15**.

Stufe 4:

3-Chlorpropylsulfonsäureester des 1,13-Bis(phenoxy)-5,9-dioxa-7-ethyl-7-(hydroxymethyl)-2,4,10,12-tetraoxotridecans (**16**)

3,2 g (11,6 mmol) der vorstehend beschriebenen Verbindung **15** werden mit 6,46 g (23,2 mmol) 5-(2-Phenoxy-1-hydroxyethyliden)-2,2-dimethyl-1,3-dioxan-4,6-dion in 25 ml Aceton eingetragen und langsam erwärmt. Ab etwa 60 °C beginnt eine starke Kohlendioxid-Entwicklung. Die Lösung wird für 2 h am Rückfluß gehalten und anschließend das Lösungsmittel im Vakuum abdestilliert. Nach Abkühlung der Reaktionsmischung verbleibt ein rotschwarzes Öl, das - neben anderen Produkten - den gewünschten β-Ketoester-Sulfonsäureester **16** enthält. Er wird ohne weitere Reinigung als Ausgangsprodukt für die nächste Stufe verwendet.

Stufe 5:

3-Chlorpropylsulfonsäureester des 1,13-Bis(phenoxy)-3,11-diazo-5,9-dioxa-7-ethyl-7-(hydroxymethyl)-2,4,10,12-tetraoxotridecans (**17**)

Die vorstehend beschriebene Verbindung **16** wird in der anfallenden Menge in 50 ml Acetonitril gelöst und auf 0°C gekühlt. Zu der erkalteten Lösung werden unter Rühren 2,76 g (14 mmol) Tosylazid hinzugefügt und anschließend 3,1 g (30 mmol) Triethylamin derart zugetropft, daß die Temperatur nicht über 5°C ansteigt. Die Mischung wird 30 min bei dieser Temperatur und anschließend 2 h bei Raumtemperatur gerührt. Die Mischung wird bei 0°C mit 2,16 g (9,5 mmol) 4-Carboxyphenylsulfonylazid versetzt und 15 min gerührt. Nach 1 h bei Raumtemperatur wird das Lösungsmittel im Vakuum abdestilliert, der Rückstand in Dichlormethan aufgenommen und dreimal mit je 100 ml 20%iger wäßriger Kaliumhydroxid-Lösung gewaschen. Anschließend wäscht man mit Wasser neutral und trocknet die organische Phase mit Natriumsulfat. Nach dem Einengen verbleibt ein orangerotes Öl, das durch Anlegen eines Hochvakuums von Lösungsmittelresten weitgehend befreit wird. Nach einer Säulenchromatographie an Kieselgel mit Dichlormethan/Hexan (1:1) als Laufmittel erhält man das nahezu farblose Produkt **17**, welches folgende Zusammensetzung aufweist.

| | | | | | |
|---|---|---|---|---|---|
| $C_{30}H_{31}ClN_4O_{11}S$ (MG 691,1) | Ber.: | C 52,1 % | H 4,5 % | N 8,1 % | S 4,6 % |
| | Gef.: | C 51,9 % | H 4,7 % | N 8,0 % | S 4,4 % |

IR (Film): 2.144 $cm^{-1}$ ($C=N_2$)

Beispiel 5

Herstellung eines Isomerengemischs zweier trifunktioneller Glycerinester der allgemeinen Formel I, die je zwei α-Diazo-β-ketoester-Einheiten und eine Sulfonsäureester-Einheit enthalten: 4-(Trifluormethyl)-phenylsulfonsäureester des 3,10-Diazo-5,8-dioxa-1,12-diphenyl-6-(hydroxymethyl)-2,4,9,11-tetraoxododecans und des 3,11-Diazo-5,9-dioxa-1,13-diphenyl-7-hydroxy-2,4,10,12-tetraoxotridecans (**22**)

Stufe 1:

Isomerengemisch aus 2,2-Dimethyl-4-(hydroxymethyl)-1,3-dioxolan und 2,2-Dimethyl-5-hydroxy-1,3-dioxan (**18**)

Zu 9,21 g (0,10 mol) Glycerin in 80 ml Aceton gibt man 0,25 g p-Toluolsulfonsäure und erhitzt zum Rückfluß. Nach 4,5 h neutralisiert man die noch heiße Lösung durch Zugabe von 0,75 g Kaliumcarbonat und rührt weitere 30 min; anschließend läßt man über Nacht bei Raumtemperatur stehen. Die trübe Mischung wird über Kieselgur filtriert und das Lösungsmittel im Vakuum abdestilliert. Es verbleiben etwa 30 ml eines trüben, öligen Rückstands, der durch Destillation im Hochvakuum gereinigt wird. Man erhält 7,4 g eines Gemischs der isomeren Alkohole **18** als farbloses Öl.

Stufe 2:

4-(Trifluormethyl)phenylsulfonsäureester des 2,2-Dimethyl-4-(hydroxymethyl)-1,3-dioxolans und des 2,2-Dimethyl-5-hydroxy-1,3-dioxans (**19**)

6,00 g (45,4 mmol) des vorstehend beschriebenen Isomerengemischs **18** werden in 20,2 g (0,20 mol) N-Methylmorpholin eingetragen, auf 0°C gekühlt und gerührt. Nach 15 min werden 12,2 g (50 mmol) 4-(Trifluormethyl)phenylsulfonsäurechlorid zur Lösung gegeben. Man rührt 3 h bei 0°C und versetzt mit 150 ml Eiswasser/100 ml Dichlormethan. Die organische Phase wird abgetrennt, die wäßrige Phase mit Dichlormethan nachextrahiert. Die vereinigten organischen Phasen werden fünfmal mit je 100 ml Wasser gewaschen, mit Natriumsulfat getrocknet und das Lösungsmittel im Vakuum abdestilliert. Es verbleiben 13,6

g eines beigefarbenen Öls, das partiell kristallisiert. Das Rohprodukt, das den gewünschten Sulfonsäureester **19** enthält, wird ohne weitere Reinigung als Ausgangsprodukt für die Stufe 3 verwendet.

Stufe 3:

Isomere 4-(Trifluormethyl)phenylsulfonsäureester des Glycerins (**20**)

8,0 g (23,5 mmol) der in Stufe 2 hergestellten, isomeren Sulfonsäureester **19** werden in 100 ml Methanol eingetragen und es wird so lange 3 N Salzsäure zugetropft, bis der pH-Wert kleiner als fünf ist. Anschließend gibt man 0,50 g p-Toluolsulfonsäure zu, rührt 3 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert und es verbleibt ein gelboranger, öliger Rückstand. Durch Säulenchromatographie an Kieselgel mit Dichlormethan als Laufmittel erhält man 3,1 g farbloses Öl **20**.

Stufe 4:

4-(Trifluormethyl)phenylsulfonsäureester des 5,8-Dioxa-1,12-diphenyl-6-(hydroxymethyl)-2,4,9,11-tetra oxododecans und des 5,9-Dioxa-1,13-diphenyl-7-hydroxy-2,4,10,12-tetraoxotridecans (**21**)

2,1 g (7,0 mmol) der isomeren Glycerinsulfonsäureester **20** werden mit 3,7 g (14 mmol) 5-(1-Hydroxy-2-phenylethyliden)-2,2-dimethyl-1,3-dioxan-4,6-dion in 20 ml Aceton eingetragen und langsam erwärmt. Ab etwa 60 °C beginnt eine starke Kohlendioxid-Entwicklung. Die Lösung wird für 2,5 h am Rückfluß gehalten und anschließend das Lösungsmittel im Vakuum abdestilliert. Nach Abkühlung der Reaktionsmischung verbleibt ein schwarzbraunes Öl, das praktisch ausschließlich aus den gewünschten β-Ketoester-Sulfonsäureestern **21** besteht. Dieses Isomerengemisch wird ohne weitere Reinigung als Ausgangsprodukt für die nächste Stufe verwendet.

Stufe 5:

4-(Trifluormethyl)phenylsulfonsäureester des 3,10-Diazo-5,8-dioxa-1,12-diphenyl-6-(hydroxymethyl)-2,4,9,11-tetraoxododecans und des 3,11-Diazo-5,9-dioxa-1,13-diphenyl-7-hydroxy-2,4,10,12-tetraoxotridecans (**22**)

Das vorstehend beschriebene Isomerengemisch **21** wird in der anfallenden Menge in 35 ml Acetonitril gelöst und auf 0 °C gekühlt. Zu der erkalteten Lösung werden unter Rühren 2,17 g (11 mmol) Tosylazid hinzugefügt und anschließend 2,0 g (20 mmol) Triethylamin derart zugetropft, daß die Temperatur nicht über 5 °C ansteigt. Die Mischung wird 1 h bei dieser Temperatur und anschließend 2 h bei Raumtemperatur gerührt. Bei 0 °C wird mit 0,68 g (3,0 mmol) 4-Carboxyphenylsulfonylazid versetzt und 30 min gerührt. Nach 1 h bei Raumtemperatur wird das Lösungsmittel im Vakuum abdestilliert und der Ansatz über Nacht bei - 20 °C aufbewahrt. Es wird auf Raumtemperatur erwärmt, der Rückstand in Dichlormethan aufgenommen und dreimal mit je 100 ml 15%iger wäßriger Kaliumhydroxid-Lösung gewaschen. Anschließend wäscht man mit Wasser neutral und trocknet die organische Phase mit Magnesiumsulfat. Nach dem Einengen verbleibt ein schwarzrotes Öl, das durch Anlegen eines Hochvakuums von Lösungsmittelresten weitgehend befreit wird. Nach einer Säulenchromatographie an Kieselgel mit Dichlormethan als Laufmittel erhält man 1,2 g nahezu farbloses Produkt **22**, welches folgende Zusammensetzung aufweist.

| | | | | | |
|---|---|---|---|---|---|
| $C_{30}H_{23}F_3N_4O_9S$ (MG 672,6) | Ber.:<br>Gef.: | C 53,6 %<br>C 53,9 % | H 3,5 %<br>H 3,8 % | N 8,3 %<br>N 8,0 % | S 4,8 %<br>S 4,7 % |

IR (KBr): 2.137 $cm^{-1}$ ($C=N_2$)

Beispiel 6

Herstellung eines trifunktionellen Esters mit zwei α-Diazo-β-ketoester-Einheiten und einer Sulfonsäureester-Einheit der allgemeinen Formel I: p-Toluolsulfonsäureester der 1,3-Bis(5-diazo-4,6-dioxo-7-methyl-3-oxaoctyl)-5-(2-hydroxyethyl)cyanursäure (**27**)

Stufe 1:

1,3-Bis[3-oxa-3-(tetrahydropyranyl)propyl]-5-(2-hydroxyethyl)cyanursäure (**23**)

20,9 g (80 mmol) 1,3,5-Tris(2-hydroxyethyl)cyanursäure werden zusammen mit 20,2 g (0,24 mol) 3,4-Dihydro-2H-pyran in 750 ml Dichlormethan eingetragen. Man versetzt mit 4,02 g (16 mmol) Pyridinium-(toluol-4-sulfonat) und rührt 5 h bei Raumtemperatur. Anschließend wird das Lösungsmittel im Vakuum abdestilliert und die eingeengte Mischung in Ether aufgenommen. Man wäscht zweimal mit je 200 ml halbgesättigter wäßriger Natriumchlorid-Lösung und zweimal mit 150 ml Wasser. Trocknen mit Magnesiumsulfat und Abdestillieren des Lösungsmittels im Vakuum ergibt einen nahezu farblosen Rückstand, der u. a. den Tetrahydropyranylether **23** enthält. 22 g dieser Verbindung werden durch Säulenchromatographie an Kieselgel mit Dichlormethan als Laufmittel von den anderen Produkten abgetrennt.

Stufe 2:

p-Toluolsulfonsäureester der 1,3-Bis[3-oxa-3-(tetrahydropyranyl)-propyl]-5-(2-hydroxyethyl)cyanursäure (**24**)

8,59 g (20 mmol) der vorstehend beschriebenen Verbindung **23** werden mit 4,6 g (45 mmol) N-Methylmorpholin in 50 ml Acetonitril eingetragen, auf 0 °C gekühlt und gerührt. Nach 30 min werden 4,19 g (22 mmol) p-Toluolsulfonsäurechlorid zur Lösung gegeben. Man rührt 4 h bei 0 °C und destilliert das Lösungsmittel im Vakuum ab. Der verbleibende, ölige Rückstand wird mit 150 ml Wasser/ 100 ml Dichlormethan versetzt und die organische Phase abgetrennt. Die wäßrige Phase wird mit Dichlormethan nachextrahiert und die vereinigten organischen Phasen werden dreimal mit je 150 ml halbgesättigter Kaliumdihydrogenphosphat-Lösung gewaschen. Anschließend wäscht man mit Wasser neutral, trocknet mit Natriumsulfat und destilliert das Lösungsmittel im Vakuum ab. Es verbleiben 10 g eines gelblichen Öls, das den geschützten Sulfonsäureester **24** enthält. Der rohe Sulfonsäureester **24** wird ohne Reinigung in der nächsten Stufe weiter umgesetzt.

Stufe 3:

p-Toluolsulfonsäureester der 1,3,5-Tris(2-hydroxyethyl)cyanursäure (**25**)

8,75 g (15 mmol) des in Stufe 2 hergestellten Tetrahydropyranylether-Sulfonsäureesters **24** werden in 250 ml eines Lösungsmittelgemischs - bestehend aus Essigsäure, Tetrahydrofuran und Wasser (3:2:1) - eingetragen und 8 h bei 50 °C gerührt. Anschließend versetzt man mit methanolischer Kaliumhydroxid-Lösung bis die Mischung schwach alkalisch (pH ≈ 8 bis 9) reagiert. Das Lösungsmittel wird im Vakuum abdestilliert und der Rückstand in 150 ml Dichlormethan aufgenommen. Man wäscht zweimal mit je 80 ml halbgesättigter wäßriger Natriumchlorid-Lösung und mit 100 ml Wasser, trocknet mit Natriumsulfat und destilliert das Lösungsmittel im Vakuum ab. Es verbleibt ein gelboranges Öl, das durch Säulenchromatographie an Kieselgel mit Dichlormethan/Essigester als Laufmittel aufgetrennt wird. Man erhält 4,3 g farbloses Öl **25**, das nach kurzer Zeit kristallisiert.

Stufe 4:

p-Toluolsulfonsäureester der 1,3-Bis(4,6-dioxo-7-methyl-3-oxaoctyl)-5-(2-hydroxyethyl)cyanursäure (**26**)

4,0 g (9,6 mmol) der vorstehend beschriebenen Verbindung **25** werden mit 4,11 g (19,2 mmol) 5-(1-Hydroxy-2-methylpropyliden)-2,2-dimethyl-1,3-dioxan-4,6-dion in 20 ml Aceton eingetragen und langsam erwärmt. Ab etwa 60 °C beginnt eine starke Kohlendioxid-Entwicklung. Die Lösung wird für 2,5 h am Rückfluß gehalten und das Lösungsmittel im Vakuum abdestilliert. Nach Abkühlung der Reaktionsmischung verbleibt ein tiefrotes Öl, das praktisch ausschließlich aus dem gewünschten $\beta$-Ketoester-Sulfonsäureester **26** besteht. Er wird ohne weitere Reinigung als Ausgangsprodukt für die nächste Stufe verwendet.

Stufe 5:

p-Toluolsulfonsäureester der 1,3-Bis(5-diazo-4,6-dioxo-7-methyl-3-oxaoctyl)-5-(2-hydroxyethyl)cyanursäure (**27**)

Die vorstehend beschriebene Verbindung **26** wird in der anfallenden Menge in 50 ml Acetonitril gelöst und auf 0 °C gekühlt. Zu der erkalteten Lösung werden unter Rühren 2,37 g (12 mmol) Tosylazid hinzugefügt und anschließend 2,5 g (25 mmol) Triethylamin derart zugetropft, daß die Temperatur nicht über 5 °C ansteigt. Die Mischung wird 60 min bei dieser Temperatur gerührt; anschließend wird auf Raumtemperatur erwärmt. Nach 2 h ist in der Mischung dünnschichtchromatographisch (Kieselgel, Laufmittel: Dichlormethan) kein Tosylazid mehr nachweisbar. Die Mischung wird bei 0 °C mit 1,70 g (7,5 mmol) 4-Carboxyphenylsulfonylazid versetzt und 30 min gerührt. Nach weiteren 2 h bei Raumtemperatur wird das Lösungsmittel im Vakuum abdestilliert. Der Rückstand wird in Dichlormethan aufgenommen und dreimal mit je 200 ml 10%iger wäßriger Kaliumhydroxid-Lösung gewaschen. Anschließend wäscht man mit Wasser neutral und trocknet die organische Phase mit Natriumsulfat. Nach dem Einengen verbleibt ein rotes Öl, das durch Anlegen eines Hochvakuums von Lösungsmittelresten weitgehend befreit wird. Nach einer Säulenchromatographie an Kieselgel mit Dichlormethan/Essigester als Laufmittel erhält man 5,4 g farblosen $\alpha$-Diazo-$\beta$-ketoester-Sulfonsäureester**27**, welcher folgende Zusammensetzung aufweist.

| | | | | | |
|---|---|---|---|---|---|
| $C_{28}H_{33}N_7O_{12}S$ (MG 691,7) | Ber.:<br>Gef.: | C 48,6 %<br>C 49,0 % | H 4,8 %<br>H 4,9 % | N 14,2 %<br>N 13,2 % | S 4,6 %<br>S 4,6 % |

IR (KBr): 2.137 $cm^{-1}$ ($C=N_2$)

Beispiel 7

Herstellung eines trifunktionellen Esters mit zwei $\alpha$-Diazo-$\beta$-ketoester-Einheiten und einer Sulfonsäureester-Einheit der allgemeinen Formel I: Isopropylsulfonsäureester des 1,1-Bis[4-(6-diazo-1,4-dioxa-5,7-dioxononyl)phenyl]-1-[4-(3-hydroxy-1-oxapropyl)phenyl]ethans (**30**)

Stufe 1:

Isopropylsulfonsäureester des 1,1,1-Tris[4-(3-hydroxy-1-oxapropyl)phenyl]ethans (**28**)

21,9 g (50 mmol) 1,1,1-Tris[4-(3-hydroxy-1-oxapropyl)phenyl]ethan werden mit 10,6 g (105 mmol) N-Methylmorpholin in 100 ml Acetonitril eingetragen, auf 0 °C gekühlt und gerührt. Nach 30 min werden 7,56 g (53 mmol) Isopropylsulfonsäurechlorid zur Lösung gegeben. Man rührt 6 h bei 0 °C und bewahrt die Mischung über Nacht bei -20 °C auf. Nach Erwärmen auf Raumtemperatur wird das Lösungsmittel im Vakuum abdestilliert. Der verbleibende, ölige Rückstand wird mit 300 ml Wasser und mit 200 ml Dichlormethan versetzt. Die organische Phase wird abgetrennt, die wäßrige Phase mit Dichlormethan nachextrahiert. Die vereinigten organischen Phasen werden dreimal mit Wasser neutral gewaschen, mit Natriumsulfat getrocknet und das Lösungsmittel im Vakuum abdestilliert. Reste des Lösungsmittels werden im Hochvakuum nahezu vollständig abdestilliert und es verbleiben 26 g einer gelben, viskosen Masse, die als Hauptprodukt den Monosulfonsäureester **28** enthält. Dieser wird durch präparative Mitteldruck-Flüssigkeitschromatographie an Kieselgel (Laufmittel: Petrolether/ Essigester) abgetrennt. Man erhält 14,6 g farblosen, kristallinen Monosulfonsäureester **28**.

Stufe 2:

Isopropylsulfonsäureester des 1,1-Bis[4-(1,4-dioxa-5,7-dioxononyl)phenyl]-1-[4-(3-hydroxy-1-oxapropyl)-phenyl]ethans (**29**)

2,5 g (4,6 mmol) der vorstehend beschriebenen Verbindung **28** werden mit 1,49 g (11,5 mmol) 3-Oxovaleriansäuremethylester langsam erwärmt und für 2,5 h am Rückfluß gehalten. Anschließend wird das gebildete Methanol und noch vorhandener 3-Oxovaleriansäuremethylester im Vakuum abdestilliert. Nach Abkühlung der Reaktionsmischung verbleibt ein tiefrotes Öl, das praktisch ausschließlich aus dem gewünschten $\beta$-Ketoester-Sulfonsäureester **29** besteht. Er wird ohne weitere Reinigung für die nächste Stufe

verwendet.

Stufe 3:

Isopropylsulfonsäureester des 1,1-Bis[4-(6-diazo-1,4-dioxa-5,7-dioxononyl)phenyl]-1-[4-(3-hydroxy-1-oxapropyl)phenyl]ethans (**30**)

Die vorstehend beschriebene Verbindung **29** wird in der in Stufe 2 anfallenden Menge in 50 ml Acetonitril gelöst und auf 0 °C gekühlt. Zu der erkalteten Lösung werden unter Rühren 1,48 g (7,5 mmol) Tosylazid und anschließend 1,5 g (15 mmol) Triethylamin hinzugefügt. Die Mischung wird 1 h bei dieser Temperatur gerührt und anschließend 2 h bei Raumtemperatur. Bei 0 °C werden 0,39 g (1,7 mmol) 4-Carboxyphenylsulfonylazid zugegeben und 30 min gerührt. Nach weiteren 2 h bei Raumtemperatur wird das Lösungsmittel im Vakuum abdestilliert. Der Rückstand wird in Dichlormethan aufgenommen und mehrmals mit je 10%iger wäßriger Kaliumhydroxid-Lösung gewaschen. Anschließend wäscht man mit Wasser neutral und trocknet die organische Phase mit Natriumsulfat. Nach dem Einengen verbleibt ein schwarzrotes Öl, das durch Anlegen eines Hochvakuums von Lösungsmittelresten weitgehend befreit wird. Nach einer Säulenchromatographie an Kieselgel mit Dichlormethan als Laufmittel erhält man das nahezu farblose Produkt **30**, welches folgende Zusammensetzung aufweist.

| | | | | | |
|---|---|---|---|---|---|
| $C_{39}H_{44}N_4O_{12}S$ (MG 792,9) | Ber.:<br>Gef.: | C 59,1 %<br>C 58,7 % | H 5,6 %<br>H 5,6 % | N 7,1 %<br>N 7,4 % | S 4,0 %<br>S 4,4 % |

IR (KBr): 2.138 $cm^{-1}$ ($C=N_2$)

Beispiel 8

Herstellung eines trifunktionellen Esters mit zwei $\alpha$-Diazo-$\beta$-ketoester-Einheiten und einer Sulfonsäureester-Einheit der allgemeinen Formel I: 3-Phenylpropylsulfonsäureester des 1,11-Diadamantyl-2,10-diazo-4,8-dioxa-6-(hydroxymethyl)-6-methyl-1,3,9,11-tetraoxoundecans (**35**)

Stufe 1:

5-(Hydroxymethyl)-2,2,5-trimethyl-1,3-dioxan (**31**)

Zu 96,2 g (0,80 mol) 2-(Hydroxymethyl)-2-methyl-1,3-propandiol in 600 ml Aceton gibt man 1,0 g p-Toluolsulfonsäure und erhitzt zum Rückfluß. Nach 5 h neutralisiert man die noch heiße Lösung durch Zugabe von 3,0 g Kaliumcarbonat und rührt weitere 30 min; anschließend läßt man über Nacht bei Raumtemperatur stehen. Die Mischung wird über Kieselgur filtriert und das Lösungsmittel im Vakuum abdestilliert. Es verbleiben etwa 180 ml eines trüben, öligen Rückstands, der durch Destillation im Hochvakuum gereinigt wird. Man erhält 78 g des Alkohols **31** als farbloses, viskoses Öl.

Stufe 2:

3-Phenylpropylsulfonsäureester des 5-(Hydroxymethyl)-2,2,5-trimethyl-1,3-dioxans (**32**)

10,4 g (65 mmol) des partiell geschützten Alkohols **31** werden in 15,1 g (0,15 mol) N-Methylmorpholin eingetragen, auf 0 °C gekühlt und gerührt. Nach 15 min werden 14,3 g (70 mmol) 3-Phenylpropylsulfonsäurechlorid zur Lösung gegeben. Man rührt 4 h bei 0 °C und destilliert das Lösungsmittel im Vakuum ab. Der verbleibende Rückstand wird mit 500 ml Wasser und mit 250 ml Dichlormethan versetzt. Die organische Phase wird abgetrennt, die wäßrige Phase mit Dichlormethan nachextrahiert. Die vereinigten organischen Phasen werden dreimal mit je 100 ml halbgesättigter Kaliumdihydrogenphosphat-Lösung und mit Wasser neutral gewaschen, mit Natriumsulfat getrocknet und das Lösungsmittel im Vakuum abdestilliert. Es verbleiben 14 g eines gelblichen Öls, das den gewünschten Sulfonsäureester **32** enthält. Dieser wird ohne weitere Reinigung als Ausgangsprodukt für die nächste Stufe verwendet.

Stufe 3:

3-Phenylpropylsulfonsäureester des 2-(Hydroxymethyl)-2-methyl-1,3-propandiols (**33**)

10,0 g (30,4 mmol) des in Stufe 2 hergestellten, geschützten Sulfonsäureesters **32** werden in 250 ml Methanol eingetragen. Es wird so lange 3 N Salzsäure zugetropft, bis der pH-Wert kleiner als fünf ist. Anschließend gibt man 0,40 g p-Toluolsulfonsäure zu, rührt 6 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert und der verbleibende Rückstand durch Säulenchromatographie an Kieselgel mit Dichlormethan als Laufmittel aufgetrennt. Man erhält 5,4 g farbloses Öl **33**, das nach wenigen Stunden kristallisiert.

Stufe 4:

3-Phenylpropylsulfonsäureester des 1,11-Diadamantyl-4,8-dioxa-6-(hydroxymethyl)-6-methyl-1,3,9,11-tetraoxoundecans (**34**)

3,1 g (10,7 mmol) der vorstehend beschriebenen Verbindung **33** werden mit 6,59 g (21,5 mmol) 5-(1-Adamantyl-1-hydroxymethyliden)-2,2-dimethyl-1,3-dioxan-4,6-dion in 35 ml Aceton eingetragen und langsam erwärmt. Ab etwa 60 °C beginnt eine starke Kohlendioxid-Entwicklung. Die Lösung wird für 2,5 h am Rückfluß gehalten und anschließend das Lösungsmittel im Vakuum abdestilliert. Nach Abkühlung der Reaktionsmischung verbleibt ein öliger Rückstand, der den tiefroten β-Ketoester-Sulfonsäureester **34** enthält und ohne weitere Reinigung in der nächsten Stufe eingesetzt wird.

Stufe 5:

3-Phenylpropylsulfonsäureester des 1,11-Diadamantyl-2,10-diazo-4,8-dioxa-6-(hydroxymethyl)-6-methyl-1,3,9,11-tetraoxoundecans (**35**)

Die vorstehend beschriebene Verbindung **34** wird in der in Stufe 4 anfallenden Menge in 50 ml Acetonitril/10 ml Aceton gelöst und auf 0 °C gekühlt. Zu der erkalteten Lösung werden unter Rühren 2,76 g (14 mmol) Tosylazid hinzugefügt und anschließend 2,5 g (25 mmol) Triethylamin derart zugetropft, daß die Temperatur nicht über 5 °C ansteigt. Die Mischung wird 1 h bei dieser Temperatur gerührt und anschließend auf Raumtemperatur erwärmt. Nach 2 h wird bei O °C mit 1,70 g (7,5 mmol) 4-Carboxyphenylsulfonylazid versetzt und 15 min gerührt. Nach weiteren 2 h bei Raumtemperatur wird das Lösungsmittel im Vakuum abdestilliert. Der Rückstand wird in Dichlormethan aufgenommen und dreimal mit je 100 ml 15%iger wäßriger Kaliumhydroxid-Lösung gewaschen. Anschließend wäscht man mit Wasser neutral und trocknet die organische Phase mit Natriumsulfat. Nach dem Einengen verbleibt ein orangerotes Öl, das durch Anlegen eines Hochvakuums von Lösungsmittelresten weitgehend befreit wird. Säulenchromatographie an Kieselgel mit Dichlormethan als Laufmittel ergibt 1,2 g nahezu farblosen α-Diazo-β-ketoester-Sulfonsäureester **35**, welcher folgende Zusammensetzung aufweist.

| | | | | | |
|---|---|---|---|---|---|
| $C_{39}H_{48}N_4O_9S$ (MG 748,9) | Ber.:<br>Gef.: | C 62,6 %<br>C 62,3 % | H 6,5 %<br>H 6,3 % | N 7,5 %<br>N 7,2 % | S 4,3 %<br>S 4,3 % |

IR (KBr): 2.137 $cm^{-1}$ ($C=N_2$)

<u>Beispiel 9</u>

Herstellung eines trifunktionellen Esters mit zwei α-Diazo-β-ketoester-Einheiten und einer Sulfonsäureester-Einheit der allgemeinen Formel I: 4-Cyanophenylsulfonsäureester des 8,16-Diazo-2,5,10,14,-19,22-hexoxa-12-(hydroxymethyl)-12-methyl-7,9,15,17-tetraoxotrieicosans (**39**)

Stufe 1:

5-(Hydroxymethyl)-2,2,5-trimethyl-1,3-dioxan (**31**) Herstellung siehe Beispiel 8, Stufe 1;

Stufe 2:

4-Cyanophenylsulfonsäureester des 5-(Hydroxymethyl)-2,2,5-trimethyl-1,3-dioxans (**36**)

5,77 g (36 mmol) der vorstehend beschriebenen Verbindung **31** werden in 10,1 g (0,10 mol) N-Methylmorpholin eingetragen, auf 0 °C gekühlt und gerührt. Nach 10 min werden 8,07 g (40 mmol) 4-Cyanophenylsulfonsäurechlorid zur Lösung gegeben. Man rührt 5 h bei 0 °C und versetzt mit 250 ml Wasser/150 ml Dichlormethan. Die organische Phase wird abgetrennt, die wäßrige Phase mit Dichlormethan nachextrahiert. Die vereinigten organischen Phasen werden dreimal mit je 100 ml halbgesättigter Kaliumdihydrogenphosphat-Lösung und mit Wasser neutral gewaschen, mit Natriumsulfat getrocknet und das Lösungsmittel im Vakuum abdestilliert. Es verbleiben 9,8 g schwach gelbliche Kristalle, die praktisch ausschließlich aus dem gewünschten Sulfonsäureester **36** bestehen. Er wird ohne weitere Reinigung als Ausgangsprodukt für die nächste Stufe verwendet.

Stufe 3:

4-Cyanophenylsulfonsäureester des 2-(Hydroxymethyl)-2-methyl-1,3-propandiolS (**37**)

9,76 g (30 mmol) des in Stufe 2 hergestellten, geschützten Sulfonsäureesters **36** werden in 150 ml Methanol eingetragen und es wird so lange 3 N Salzsäure zugetropft, bis der pH-Wert kleiner als fünf ist. Anschließend gibt man 0,50 g p-Toluolsulfonsäure zu, rührt 4,5 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert und der verbleibende Rückstand wird durch Säulenchromatographie an Kieselgel mit Dichlormethan als Laufmittel aufgetrennt. Man erhält 5,2 g kristallines Produkt **37**.

Stufe 4:

4-Cyanophenylsulfonsäureester des 2,5,10,14,19,22-Hexoxa-12-(hydroxymethyl)-12-methyl-7,9,15,17-tetraoxotrieicosans (**38**)

2,85 g (10,0 mmol) der vorstehend beschriebenen Verbindung **37** werden mit 5,21 g (20,0 mmol) 5-(3,6-Dioxa-1-hydroxyheptyliden)-2,2-dimethyl-1,3-dioxan-4,6-dion in 20 ml Aceton eingetragen und langsam erwärmt. Ab etwa 60 °C beginnt eine starke Kohlendioxid-Entwicklung. Die Lösung wird für 2,5 h am Rückfluß gehalten und anschließend das Lösungsmittel abdestilliert. Nach Abkühlung der Reaktionsmischung verbleibt ein schwarzbraunes Öl, das den β-Ketoester-Sulfonsäureester **38** als Hauptprodukt enthält. Dieses wird ohne weitere Reinigung in Stufe 5 eingesetzt.

Stufe 5:

4-Cyanophenylsulfonsäureester des 8,16-Diazo-2,5,10,14,19,22-hexoxa-12-(hydroxymethyl)-12-methyl-7,9,15,17-tetraoxotrieicosans (**39**)

Die vorstehend beschriebene Verbindung **38** wird in der anfallenden Menge in 50 ml Acetonitril gelöst und auf 0 °C gekühlt. Zu der erkalteten Lösung werden unter Rühren 2,37 g (12 mmol) Tosylazid hinzugefügt und anschließend 2,5 g (25 mmol) Triethylamin derart zugetropft, daß die Temperatur nicht über 5 °C ansteigt. Die Mischung wird 1 h bei dieser Temperatur gerührt; anschließend wird auf Raumtemperatur erwärmt. Nach 2 h ist in der Mischung dünnschichtchromatographisch (Kieselgel, Laufmittel: Dichlormethan) kein Tosylazid mehr nachweisbar. Die Mischung wird bei 0 °C mit 1,82 g (8,0 mmol) 4-Carboxyphenylsulfonylazid versetzt und 30 min gerührt. Nach weiteren 2 h bei Raumtemperatur wird das Lösungsmittel im Vakuum abdestilliert und der Rückstand in Dichlormethan aufgenommen. Man wäscht dreimal mit je 100 ml 10%iger wäßriger Kaliumhydroxid-Lösung und dreimal mit je 80 ml Wasser, trocknet die organische Phase mit Natriumsulfat und destilliert das Lösungsmittel im Vakuum ab. Es verbleibt ein rotes Öl, das durch Anlegen eines Hochvakuums von Lösungsmittelresten weitgehend befreit wird. Nach

einer Säulenchromatographie an Kieselgel mit Dichlormethan als Laufmittel erhält man den nahezu farblosen α-Diazo-β-ketoester-Sulfonsäureester **39**, welcher folgende Zusammensetzung aufweist.

| | | | | | |
|---|---|---|---|---|---|
| $C_{26}H_{31}N_5O_{13}S$ (MG 653,6) | Ber.:<br>Gef.: | C 47,8 %<br>C 48,2 % | H 4,8 %<br>H 4,8 % | N 10,7 %<br>N 10,8 % | S 4,9 %<br>S 4,6 % |

IR (KBr): 2.143 $cm^{-1}$ ($C=N_2$)

Beispiel 10

Herstellung eines pentafunktionellen Esters mit vier α-Diazo-β-ketoester-Einheiten und einer Sulfonsäureester-Einheit der allgemeinen Formel I: 4-Bromphenylsulfonsäureester des 1,2,10,11-Tetra(4-cyclohexyl-3-diazo-2,4-dioxo-1-oxabutyl)triglycerins (**43**)

Stufe 1:

4-Bromphenylsulfonsäureester des 1,2,10,11-O,O-Di-(isopropyliden)triglycerins (**40**)

20,0 g (62,4 mmol) des kommerziell erhältlichen O,O-Di(isopropyliden)triglycerins werden in 25,2 g (0,32 mol) Pyridin eingetragen, auf 0 °C gekühlt und gerührt. Nach 20 min werden 22,4 g (87,6 mmol) 4-Bromphenylsulfonsäurechlorid zur Lösung gegeben. Man rührt 4 h bei 0 °C und 30 min bei Raumtemperatur. Der gebildete Niederschlag wird abfiltriert, mehrfach mit Wasser gewaschen und in 300 ml Dichlormethan aufgenommen. Die organische Phase wird abgetrennt, die wäßrige Phase mit Dichlormethan nachextrahiert und die vereinigten organischen Phasen werden viermal mit je 200 ml halbgesättigter Kaliumdihydrogenphosphat-Lösung gewaschen. Anschließend wäscht man mit Wasser neutral, trocknet mit Natriumsulfat und destilliert das Lösungsmittel im Vakuum ab. Es verbleiben 29 g eines gelben Öls, das teilweise kristallisiert und praktisch ausschließlich aus dem Sulfonsäureester **40** besteht. Dieser wird ohne weitere Reinigung als Ausgangsprodukt für die nächste Stufe verwendet.

Stufe 2:

4-Bromphenylsulfonsäureester des Triglycerins (**41**)

14 g (26 mmol) des in Stufe 1 hergestellten, geschützten Triglycerin-Sulfonsäuresters **40** werden in 80 ml Methanol eingetragen, und es wird so lange 3 N Salzsäure zugetropft, bis der pH-Wert kleiner als fünf ist. Anschließend gibt man 0,35 g p-Toluolsulfonsäure zu, rührt 16 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert, und es verbleibt ein Öl, das durch Säulenchromatographie an Kieselgel mit Dichlormethan/Essigester als Laufmittel aufgetrennt wird. Man erhält 9,8 g Triglycerin-6-sulfonsäureester **41** als farbloses Öl.

Stufe 3:

4-Bromphenylsulfonsäureester des 1,2,10,11-Tetra(4-cyclohexyl-2,4-dioxo-1-oxabutyl)triglycerins (**42**)

3,2 g (7,0 mmol) des vorstehend beschriebenen Triglycerin-6-sulfonsäureesters **41** werden mit 7,7 g (42 mmol) 3-Cyclohexyl-3-oxopropionsäuremethylester langsam erwärmt. Die homogene Mischung wird für 2 h am Rückfluß gehalten und anschließend das gebildete Methanol und noch vorhandener 3-Cyclohexyl-3-oxopropionsäuremethylester im Vakuum abdestilliert. Nach Abkühlung der Reaktionsmischung verbleibt ein schwarzbraunes, zähviskoses Öl, das den β-Ketoester-Sulfonsäureester **42** enthält. Dieser wird ohne weitere Reinigung in Stufe 4 eingesetzt.

Stufe 4:

4-Bromphenylsulfonsäureester des 1,2,10,11-Tetra(4-cyclohexyl-3-diazo-2,4-dioxo-1-oxabutyl)triglycerins (**43**)

Die vorstehend beschriebene Verbindung **42** wird in der anfallenden Menge in 80 ml Acetonitril gelöst und auf 0 °C gekühlt. Zu der erkalteten Lösung werden unter Rühren 3,94 g (20 mmol) Tosylazid hinzugefügt und anschließend 3,5 g (35 mmol) Triethylamin derart zugetropft, daß die Temperatur nicht über 5 °C ansteigt. Die Mischung wird 45 min bei dieser Temperatur gerührt und anschließend auf Raumtemperatur erwärmt. Nach 1,5 h ist in der Mischung dünnschichtchromatographisch (Kieselgel, Laufmittel: Dichlormethan) kein Tosylazid mehr nachweisbar. Die Mischung wird bei O °C mit 1,82 g (8,0 mmol) 4-Carboxyphenylsulfonylazid versetzt und 45 min gerührt. Nach weiteren 1,5 h bei Raumtemperatur wird das Lösungsmittel im Vakuum abdestilliert. Der Rückstand wird in Dichlormethan aufgenommen und dreimal mit je 100 ml 15%iger wäßriger Kaliumhydroxid-Lösung gewaschen. Anschließend wäscht man mit Wasser neutral und trocknet die organische Phase mit Natriumsulfat. Nach dem Einengen verbleibt ein tiefrotes Öl, das durch Anlegen eines Hochvakuums von Lösungsmittelresten weitgehend befreit wird. Nach einer Säulenchromatographie an Kieselgel mit Dichlormethan als Laufmittel erhält man 4,2 g nahezu farbloses Produkt **43**, welches folgende Zusammensetzung aufweist.

| | | | | | |
|---|---|---|---|---|---|
| $C_{51}H_{63}BrN_8O_{17}S$ (MG 1172,1) | Ber.:<br>Gef.: | C 52,3 %<br>C 51,9 % | H 5,4 %<br>H 5,6 % | N 9,6 %<br>N 9,1 % | S 2,4 %<br>S 4,6 % |

IR (KBr): 2.138 $cm^{-1}$ ($C=N_2$)

Beispiel 11

Herstellung eines Isomerengemischs zweier pentafunktioneller Ester der allgemeinen Formel I, die je vier $\alpha$-Diazo-$\beta$-ketoester-Einheiten und eine Sulfonsäureester-Einheit enthalten: n-Propylsulfonsäureester des 2-Hydroxy-1,1,3,3-tetra(4-diazo-5-cyclohexyl-3,5-dioxo-2-oxapentyl)cyclohexans und des 2-(3-Diazo-4-cyclohexyl-2,4-dioxo-1-oxabutyl)-1-(hydroxymethyl)-1,3,3-tris(4-diazo-5-cyclohexyl-3,5-dioxo-2-oxapentyl)-cyclohexans (**48**)

Stufe 1:

Isomere O,O-Diisopropylidenacetale des 2-Hydroxy1,1,3,3-tetra(hydroxymethyl)cyclohexans (**44**)

Zu 16,2 g (73,5 mmol) 2-Hydroxy-1,1,3,3-tetra(hydroxymethyl)cyclohexan in 120 ml Aceton gibt man 0,45 g p-Toluolsulfonsäure und erhitzt zum Rückfluß. Nach 5,5 h neutralisiert man die noch heiße Lösung durch Zugabe von 1,35 g Kaliumcarbonat und rührt weitere 40 min; anschließend läßt man über Nacht bei Raumtemperatur stehen. Die Mischung wird über Kieselgur filtriert und das Lösungsmittel im Vakuum abdestilliert. Es verbleiben etwa 35 ml eines öligen Rückstands, der durch Destillation im Hochvakuum gereinigt wird. Man erhält 13,2 g eines Gemischs der isomeren Alkohole **44** als farbloses Öl.

Stufe 2:

n-Propylsulfonsäureester der isomeren O,O-Diisopropylidenacetale des 2-Hydroxy-1,1,3,3-tetra-(hydroxymethyl)cyclohexans (**45**)

6,0 g (20 mmol) des vorstehend beschriebenen Isomerengemischs **44** werden in 12,1 g (0,12 mol) N-Methylmorpholin eingetragen, auf 0 °C gekühlt und gerührt. Nach 15 min werden 3,42 g (24 mmol) n-Propylsulfonsäurechlorid zur Lösung gegeben. Man rührt 3,5 h bei 0 °C und versetzt mit 150 ml Eiswasser/100 ml Dichlormethan. Die organische Phase wird abgetrennt, die wäßrige Phase mit Dichlormethan nachextrahiert. Die vereinigten organischen Phasen werden fünfmal mit je 100 ml Wasser gewaschen, mit Natriumsulfat getrocknet und das Lösungsmittel im Vakuum abdestilliert. Es verbleiben 4,3 g eines schmutzig gelben Öls, das die isomeren Sulfonsäureester **45** enthält. Das Rohprodukt wird ohne weitere Reinigung als Ausgangsprodukt für die Stufe 3 verwendet.

Stufe 3:

Isomere n-Propylsulfonsäureester des 2-Hydroxy-1,1,3,3-tetra(hydroxymethyl)cyclohexans (**46**)

4,3 g (10,6 mmol) der in Stufe 2 hergestellten, isomeren Sulfonsäureester **45** werden in 100 ml Methanol eingetragen und es wird so lange 3 N Salzsäure zugetropft, bis der pH-Wert kleiner als fünf ist. Anschließend gibt man 0,65 g p-Toluolsulfonsäure zu, rührt 3,5 h bei Raumtemperatur und versetzt die Mischung so lange mit methanolischer Kaliumhydroxid-Lösung bis sie schwach alkalisch (pH ≈ 8 bis 9) reagiert. Das Lösungsmittel wird im Vakuum abdestilliert und es verbleibt ein gelbes Öl. Durch Säulenchromatographie an Kieselgel mit Dichlormethan/Essigester als Laufmittel erhält man 3,0 g farbloses Öl **46**.

Stufe 4:

n-Propylsulfonsäureester des 2-Hydroxy-1,1,3,3-tetra-(5-cyclohexyl-3,5-dioxo-2-oxapentyl)cyclohexans und des 2-(4-Cyclohexyl-2,4-dioxo-1-oxabutyl)-1-(hydroxymethyl)-1,3,3-tris(5-cyclohexyl-3,5-dioxo-2-oxapentyl)-cyclohexans (**47**)

1,5 g (4,6 mmol) der isomeren Sulfonsäureester **46** werden mit 4,67 g (18,4 mmol) 5-(1-Cyclohexyl-1-hydroxymethyliden)-2,2-dimethyl-1,3-dioxan-4,6-dion in 20 ml Aceton eingetragen und langsam erwärmt. Ab etwa 60 °C beginnt eine starke Kohlendioxid-Entwicklung. Die Lösung wird für 2 h am Rückfluß gehalten und anschließend das Lösungsmittel im Vakuum abdestilliert. Nach Abkühlung der Reaktionsmischung verbleibt ein schwarzbraunes Öl, das praktisch ausschließlich aus den gewünschten β-Ketoester-Sulfonsäureestern **47** besteht. Dieses Isomerengemisch wird ohne weitere Reinigung als Ausgangsprodukt für die nächste Stufe verwendet.

Stufe 5:

n-Propylsulfonsäureester des 2-Hydroxy-1,1,3,3-tetra-(4-diazo-5-cyclohexyl-3,5-dioxo-2-oxapentyl)-cyclohexans und des 2-(3-Diazo-4-cyclohexyl-2,4-dioxo-1-oxabutyl)-1-(hydroxymethyl)-1,3,3-tris(4-diazo-5-cyclohexyl-3,5-dioxo-2-oxapentyl)cyclohexans (**48**)

Das vorstehend beschriebene Isomerengemisch **47** wird in der anfallenden Menge in 40 ml Acetonitril gelöst und auf 0 °C gekühlt. Zu der erkalteten Lösung werden unter Rühren 2,96 g (15 mmol) Tosylazid und anschließend 2,8 g (28 mmol) Triethylamin zugetropft. Die Mischung wird 1 h bei dieser Temperatur gerührt und anschließend 2 h bei Raumtemperatur. Bei O °C wird mit 1,0 g (4,4 mmol) 4-Carboxyphenylsulfonylazid versetzt und 45 min gerührt. Nach 1,5 h bei Raumtemperatur wird das Lösungsmittel im Vakuum abdestilliert und der Ansatz über Nacht bei - 20 °C aufbewahrt. Nach Erwärmen auf Raumtemperatur wird der Rückstand in Dichlormethan aufgenommen und dreimal mit je 120 ml 15%iger wäßriger Kaliumnydroxid-Lösung gewaschen. Anschließend wäscht man mit Wasser neutral und trocknet die organische Phase mit Natriumsulfat. Nach dem Einengen verbleibt ein dunkelrotes Öl, das durch Anlegen eines Hochvakuums von Lösungsmittelresten weitgehend befreit wird. Nach einer Säulenchromatographie an Kieselgel mit Dichlormethan als Laufmittel erhält man 1,9 g der isomeren α-Diazo-β-ketoester-Sulfonsäureester **48** als nahezu farbloses Produkt, welches folgende Zusammensetzung aufweist.

| | | | | | |
|---|---|---|---|---|---|
| $C_{49}H_{66}N_8O_{15}S$ (MG 1039,2) | Ber.: | C 56,6 % | H 6,4 % | N 10,8 % | S 3,1 % |
| | Gef.: | C 57,0 % | H 6,5 % | N 10,1 % | S 3,4 % |

IR (KBr): 2.138 $cm^{-1}$ ($C=N_2$)

Beispiel 12

Herstellung eines hexafunktionellen Esters mit vier α-Diazo-β-ketoester-Einheiten und zwei Sulfonsäureester-Einheiten der allgemeinen Formel I: 6,10-Bis(phenylsulfonsäureester) des 1,2,14,15-Tetra(4-tert-butyl-3-diazo-2,4-dioxo-1-oxabutyl)tetraglycerins (**52**)

Stufe 1:

6,10-Bis(phenylsulfonsäureester) des 1,2,14,15-O,O-Di(isopropyliden)tetraglycerins (**49**)

12,5 g (31,7 mmol) des kommerziell erhältlichen 1,2,14,15-O,O-Di(isopropyliden)tetraglycerins werden in 32 g (0,32 mol) N-Methylmorpholin eingetragen, auf 0 °C gekühlt und gerührt. Nach 30 min werden 11,8 g (67 mmol) Phenylsulfonsäurechlorid zur Lösung gegeben. Man rührt 8 h bei 0 °C, 30 min bei Raumtemperatur und versetzt die Suspension mit 300 ml Eiswasser/200 ml Dichlormethan. Die organische Phase wird abgetrennt, die wäßrige Phase mit Dichlormethan nachextrahiert und die vereinigten organischen Phasen werden zweimal mit je 150 ml halbgesättigter Kaliumdihydrogenphosphat-Lösung gewaschen. Anschließend wäscht man mit Wasser neutral, trocknet mit Natriumsulfat und destilliert das Lösungsmittel im Vakuum ab. Es verbleiben 14 g eines gelbbraunen Öls, das den geschützten Tetraglycerin-6,10-bis-(sulfonsäureester) **49** enthält. Dieser wird ohne weitere Reinigung in Stufe 2 eingesetzt.

Stufe 2:

6,10-Bis(phenylsulfonsäureester) des Tetraglycerins (**50**)

11,8 g (17,5 mmol) des in Stufe 1 hergestellten, geschützten Tetraglycerins **49** werden in 180 ml Methanol eingetragen und es wird so lange 3 N Salzsäure zugetropft, bis der pH-Wert kleiner als fünf ist. Anschließend gibt man 0,65 g p-Toluolsulfonsäure zu, rührt 14 h bei Raumtemperatur und versetzt die Mischung so lange mit methanolischer Kaliumhydroxid-Lösung bis die Mischung schwach alkalisch (pH ≈ 8 bis 9) reagiert. Das Lösungsmittel wird im Vakuum abdestilliert und es verbleibt ein Öl, das durch Säulenchromatographie an Kieselgel mit Dichlormethan/Essigester als Laufmittel aufgetrennt wird. Man erhält 5,1 g Tetraglycerin-6,10-bis(sulfonsäureester) **50** als farbloses Öl.

Stufe 3:

6,10-Bis(phenylsulfonsäureester) des 1,2,14,15-Tetra(4-tert-butyl-2,4-dioxo-1-oxabutyl)tetraglycerins (**51**)

2,6 g (4,4 mmol) des vorstehend beschriebenen Tetraglycerin-6,10-bis(sulfonsäureesters) **50** werden mit 4,0 g (17,6 mmol) 5-(2,2-Dimethyl-1-hydroxypropyliden)-2,2-dimethyl-1,3-dioxan-4,6-dion in 30 ml Aceton eingetragen und langsam erwärmt. Ab etwa 60 °C beginnt eine starke Kohlendioxid-Entwicklung. Man hält die homogene Mischung 2,5 h am Rückfluß und destilliert anschließend das Lösungsmittel im Vakuum ab. Nach Abkühlung der Reaktionsmischung verbleibt ein schwarzrotes, zähes Öl, das den β-Ketoester-Sulfonsäureester **51** enthält. Dieser wird ohne weitere Reinigung in Stufe 4 eingesetzt.

Stufe 4:

6,10-Bis(phenylsulfonsäureester) des 1,2,14,15-Tetra(4-tert-butyl-3-diazo-2,4-dioxo-1-oxabutyl)tetraglycerins (**52**)

Die vorstehend beschriebene Verbindung **51** wird in der anfallenden Menge in 80 ml Acetonitril gelöst und auf 0 °C gekühlt. Zu der erkalteten Lösung werden unter Rühren 2,56 g (13 mmol) Tosylazid hinzugefügt und anschließend 4,0 g (40 mmol) Triethylamin derart zugetropft, daß die Temperatur nicht über 5 °C ansteigt. Die Mischung wird 1 h bei dieser Temperatur und anschließend 2 h bei Raumtemperatur gerührt. Man versetzt anschließend bei O °C mit 1,14 g (5,0 mmol) 4-Carboxyphenylsulfonylazid und rührt 1,5 h. Nach weiteren 1,5 h bei Raumtemperatur wird das Lösungsmittel im Vakuum abdestilliert und der Rückstand in Dichlormethan aufgenommen. Man wäscht dreimal mit je 80 ml 20%iger wäßriger Kaliumhydroxid-Lösung und viermal mit je 60 ml Wasser neutral. Trocknen mit Natriumsulfat und Abdestillieren des Lösungsmittels ergeben ein tiefrotes Öl, das durch Anlegen eines Hochvakuums von Lösungsmittelresten weitgehend befreit wird. Nach einer Säulenchromatographie an Kieselgel mit Dichlormethan als Laufmittel erhält man 2,3 g α-Diazo-β-ketoester-Sulfonsäureester **52**, welcher folgende Zusammensetzung aufweist.

| | | | | | |
|---|---|---|---|---|---|
| $C_{52}H_{66}N_8O_{21}S_2$ (MG 1203,3) | Ber.:<br>Gef.: | C 51,9 %<br>C 52,3 % | H 5,5 %<br>H 5,7 % | N 9,3 %<br>N 8,6 % | S 5,3 %<br>S 5,4 % |

IR (KBr): 2.136 $cm^{-1}$ ($C=N_2$)

Beispiele 13-72

Es werden weitere Verbindungen der allgemeinen Formel I vorgestellt, die analog zu den bisher beschriebenen Beispielen hergestellt wurden. Wegen der Vielzahl der Verbindungen werden diese in der folgenden Tabelle hinsichtlich der in der allgemeinen Formel I beschriebenen Variationsmöglichkeiten charakterisiert. Als Analysenwert ist die quantitative Bestimmung des Stickstoffs hinreichend aussagefähig.

| Nr. | $R^1$ | $R^2$ | X | m | n | % N ber./gef. |
|---|---|---|---|---|---|---|
| 13 | tBu- | $pCH_3-C_6H_4-$ | $-CH_2-\overset{\vert}{C}H-CH_2-$ | 3 | 1 | 10,18/10,4 |
| 14 | $C_6H_{11}-$ | Ph- | dto. | 3 | 1 | 9,52/ 9,5 |
| 15 | $CH_3O\overset{\overset{O}{\Vert}}{C}C_2H_4-$ | $pBr-C_6H_4-$ | dto. | 3 | 1 | 8,30/ 8,1 |
| 16 | $CH_3\overset{\overset{O}{\Vert}}{C}C_2H_4-$ | $oNO_2-C_6H_4-$ | dto. | 3 | 1 | 11,49/11,7 |
| 17 | $PhCH_2OCH_2-$ | iPr- | dto. | 3 | 1 | 8,88/ 8,6 |
| 18 | $C_6H_{11}-$ | $pCH_3-C_6H_4-$ | $CH_3\underset{\vert}{\overset{\vert}{C}}(CH_2)_3-$ | 3 | 1 | 8,88/ 8,8 |
| 19 | nBu- | nBu- | dto. | 3 | 1 | 10,29/10,3 |
| 20 | tBu- | $Cl(CH_2)_3-$ | dto. | 3 | 1 | 9,92/ 9,7 |
| 21 | $nC_8H_{17}-$ | iPr- | dto. | 3 | 1 | 8,72/ 8,6 |
| 22 | $Ph(CH_2)_2-$ | $pNO_2-C_6H_4-$ | dto. | 3 | 1 | 11,66/11,3 |
| 23 | $C_6H_{11}-$ | $CH_3SO_2CH_2-$ | dto. | 3 | 1 | 8,86/ 8,7 |
| 24 | tBu- | $pBr-C_6H_4-$ | dto. | 3 | 1 | 8,71/ 8,9 |

| Nr. | $R^1$ | $R^2$ | X | m | n | % N ber./gef. |
|---|---|---|---|---|---|---|
| 25 | $C_6H_{11}-$ | $pCH_3-C_6H_4-$ | $CH_3CH_2C(CH_2)_3-$ | 3 | 1 | 8,69/ 8,8 |
| 26 | $C_6H_{11}-$ | $pBr-C_6H_4-$ | dto. | 3 | 1 | 7,90/ 7,9 |
| 27 | $C_6H_{11}-$ | $pCF_3-C_6H_4-$ | dto. | 3 | 1 | 8,02/ 8,3 |
| 28 | $C_6H_{11}-$ | 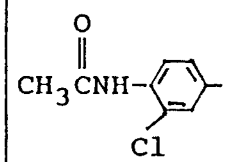 | dto. | 3 | 1 | 9,70/ 9,7 |
| 29 | tBu- | $PhCH_2-$ | dto. | 3 | 1 | 9,45/ 9,5 |
| 30 | 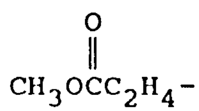 | 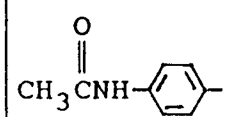 | dto. | 3 | 1 | 10,07/10,0 |
| 31 | nBu- | $pCH_3-C_6H_4-$ | $-N(CH_2CH_2)_3-$ | 3 | 1 | 12,38/12,5 |
| 32 | iBu- | $pCN-C_6H_4-$ | dto. | 3 | 1 | 14,58/14,2 |
| 33 | 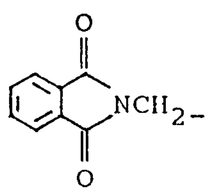 | $PhCH_2-$ | 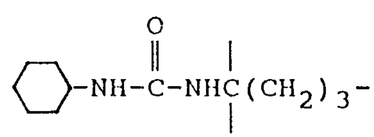 | 3 | 1 | 12,30/12,1 |

| Nr. | $R^1$ | $R^2$ | X | m | n | % N ber./gef. |
|---|---|---|---|---|---|---|
| 34 | $nC_8H_{17}-$ | $p(tBu)-C_6H_4-$ | 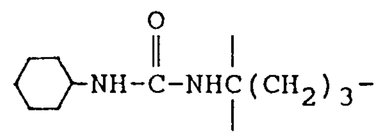 | 3 | 1 | 9,78/ 9,6 |
| 35 | $CH_3O(CH_2)_2OCH_2-$ | $Cl_3C-$ | dto. | 3 | 1 | 10,56/10,6 |
| 36 | 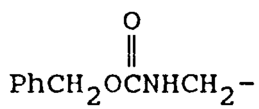 | $H_3C-$ | dto. | 3 | 1 | 12,30/12,1 |
| 37 | $nC_8H_{17}-$ | Ph- | dto. | 3 | 1 | 10,55/10,3 |
| 38 | $nC_{17}H_{35}-$ | $pCF_3-C_6H_4-$ | dto. | 3 | 1 | 7,52/ 7,6 |
| 39 | $Ph(CH_2)_2-$ | $H_3C-$ | dto. | 3 | 1 | 11,69/11,8 |
| 40 | 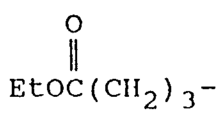 | $p(tBu)-C_6H_4-$ | dto. | 3 | 1 | 9,81/ 9,7 |
| 41 | $nC_{10}H_{21}-$ | $oNO_2-C_6H_4-$ | dto. | 3 | 1 | 10,92/10,6 |

| Nr | $R^1$ | $R^2$ | X | m | n | % N ber./gef. |
|---|---|---|---|---|---|---|
| 42 | tBu- | $pCH_3-C_6H_4-$ | 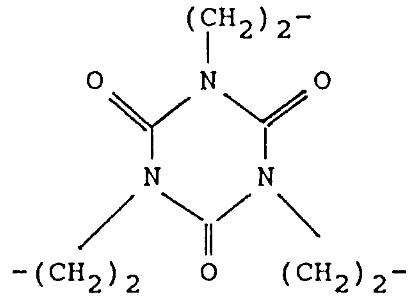 | 3 | 1 | 13,62/13,5 |
| 43 | $C_6H_{11}-$ | Ph- | dto. | 3 | 1 | 12,94/12,7 |
| 44 | $C_4H_7-$ | $H_3C-$ | dto. | 3 | 1 | 15,33/14,9 |
| 45 | 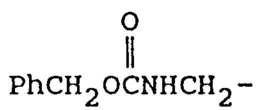 | $p(tBu)-C_6H_4-$ | dto. | 3 | 1 | 12,92/12,9 |
| 46 | $nC_6H_{13}-$ | $pCF_3-C_6H_4-$ | 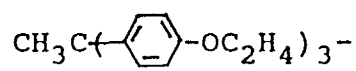 | 3 | 1 | 5,84/ 5,9 |
| 47 | $PhOCH_2-$ | $oNO_2-C_6H_4-$ | dto. | 3 | 1 | 7,15/ 7,2 |
| 48 | $C_6H_{11}-$ | $Cl(CH_2)_3-$ | dto. | 3 | 1 | 6,31/ 6,3 |
| 49 | $CH_3O(CH_2)_2OCH_2-$ | Ph- | dto. | 3 | 1 | 6,23/ 6,0 |
| 50 | $C_6H_{11}-$ | nBu- | 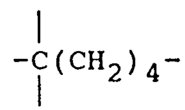 | 4 | 1 | 10,63/10,4 |
| 51 | $PhOCH_2-$ | $CH_3SO_2CH_2-$ | dto. | 4 | 1 | 9,35/ 9,7 |
| 52 | $PhCH_2-$ | $pCH_3-C_6H_4-$ | dto. | 4 | 1 | 9,90/10,2 |

| Nr. | $R^1$ | $R^2$ | X | m | n | % N ber./gef. |
|---|---|---|---|---|---|---|
| 53 | $C_6H_{11}$- | 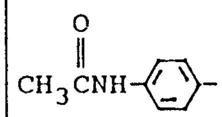 | 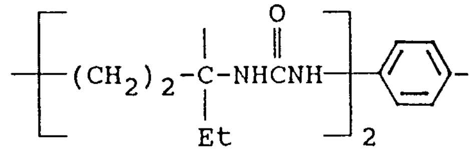 | 4 | 1 | 13,63/13,5 |
| 54 | $Ph(CH_2)_2$- | 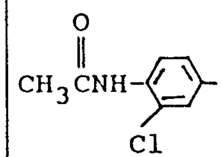 | 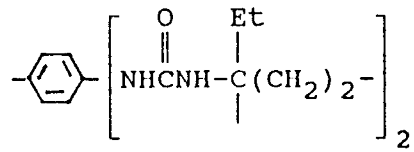 | 4 | 1 | 12,52/12,5 |
| 55 | tBu- | p(tBu)-$C_6H_4$- | 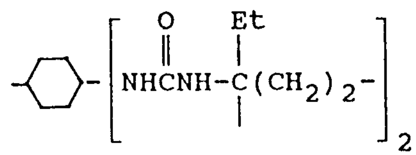 | 4 | 1 | 13,25/13,2 |
| 56 | $nC_8H_{17}$- | pH | dto. | 4 | 1 | 11,98/11,8 |
| 57 | $PhCH_2OCH_2$- | pCN-$C_6H_4$- | $-(CH_2)_2-O-CO-NH-C(CH_2)_3-$ | 4 | 1 | 10,95/10,7 |
| 58 | $C_6H_{11}$- | $pNO_2-C_6H_4$- | dto. | 4 | 1 | 12,06/12,0 |
| 59 | 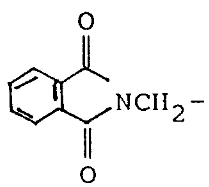 | pBr-$C_6H_4$- | $-(CH_2)_2N-CH_2CH_2-N(CH_2)_2-$ | 4 | 1 | 12,59/12,2 |
| 60 | iBu- | $pCF_3-C_6H_4$- | dto. | 4 | 1 | 12,44/12,3 |

| Nr. | $R^1$ | $R^2$ | X | m | n | % N ber./gef. |
|---|---|---|---|---|---|---|
| 61 | $C_6H_{11}-$ | $Cl(CH_2)_3-$ | 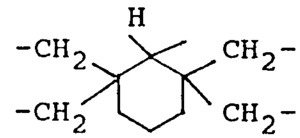 | 5 | 1 | 10,44/10,1 |
| 62 | tBu- | $pCH_3-C_6H_4-$ | dto. | 5 | 1 | 10,40/11,0 |
| 63 | $PhOCH_2-$ | $H_3C-$ | dto. | 5 | 1 | 10,12/10,5 |
| 64 | $C_6H_{11}-$ | Ph- | 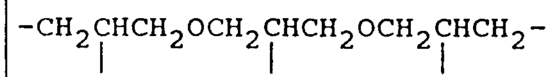 | 5 | 1 | 10,25/10,5 |
| 65 | $C_6H_{11}-$ | $pCH_3-C_6H_4-$ | dto. | 5 | 1 | 10,12/10,1 |
| 66 | $C_6H_{11}-$ | 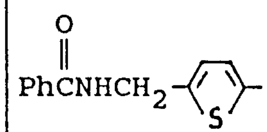 | dto. | 5 | 1 | 10,23/ 9,8 |
| 67 | $C_6H_{11}-$ | $H_3C-$ | 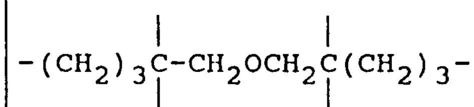 | 6 | 2 | 9,98/10,1 |
| 68 | tBu- | $H_3C-$ | dto. | 6 | 2 | 11,00/11,0 |
| 69 | $C_6H_{11}-$ | $pCH_3-C_6H_4-$ | 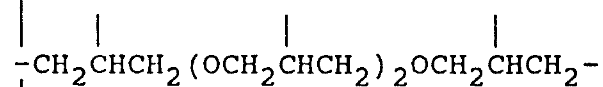 | 6 | 2 | 8,49/ 8,2 |
| 70 | $C_6H_{11}-$ | $pBr-C_6H_4-$ | 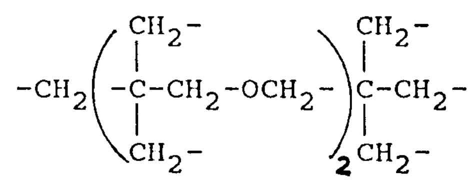 | 8 | 2 | 9,81/ 9,6 |

| Nr. | $R^1$ | $R^2$ | X | m | n | % N ber./gef. |
|---|---|---|---|---|---|---|
| 71 | $C_6H_{11}$- | iPr- | 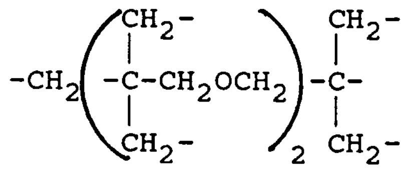 | 8 | 2 | 11,30/11,0 |
| 72 | $pCF_3-C_6H_4-$ | $pCF_3-C_6H_4-$ | " | 8 | 3 | 7,76/ 7,4 |

o = ortho (1,2-disubstituiert)
p = para (1,4-disubstituiert)

n = unverzweigte Alkylkette
i = isoverzweigte Alkylkette
t = tertiärverzweigte Alkylkette

Et = Ethyl
Bu = Butyl
Pr = Propyl
Ph = Phenyl

$C_6H_{11}$ = Cyclohexyl
$C_4H_7$ = Cyclobutyl

Im Folgenden sind die Produkte aus den Beispielen 1 bis 12, die Verbindungen 5, 9, 13, 17, 22, 27, 30, 35, 39, 43, 48 und 52 als Formelbild dargestellt.

Beispiel 1, Verbindung 5

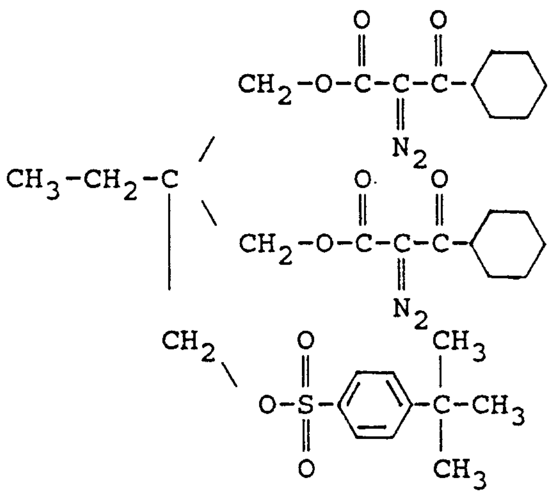

Beispiel 2, Verbindung 9

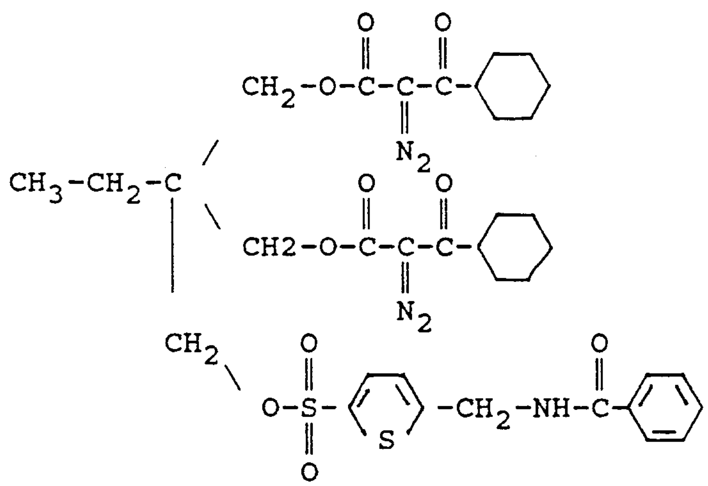

Beispiel 3, Verbindung 13

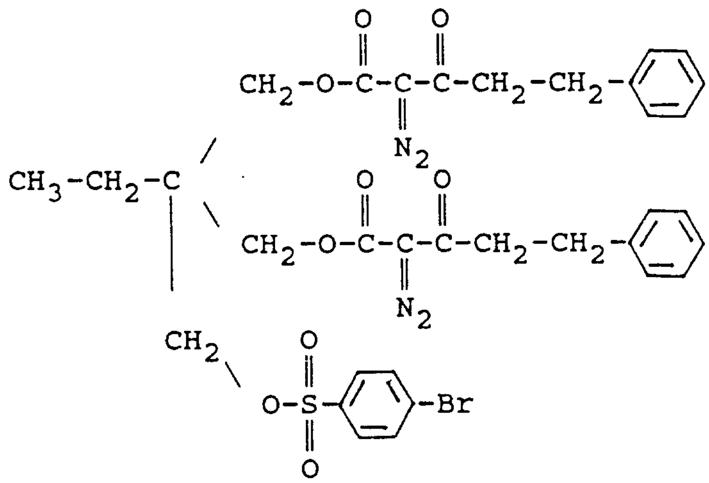

Beispiel 4, Verbindung 17

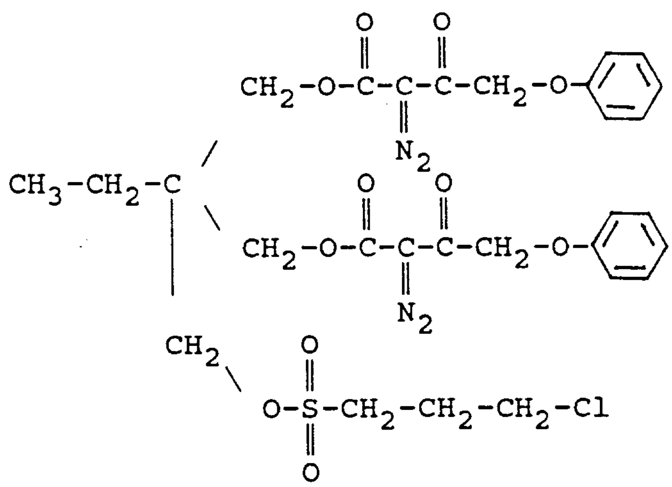

Beispiel 5, Verbindungen 22

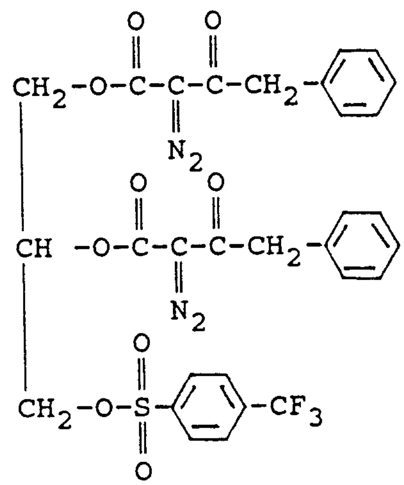

Isomerengemisch

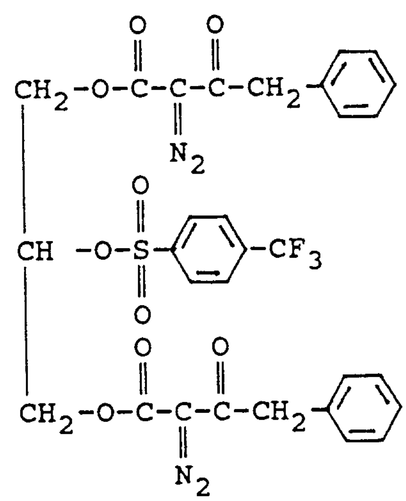

Beispiel 6, Verbindung 27

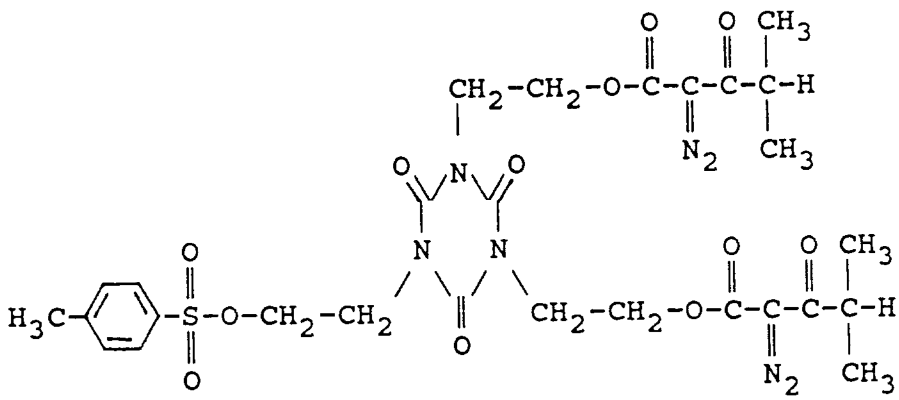

Beispiel 7, Verbindung 30

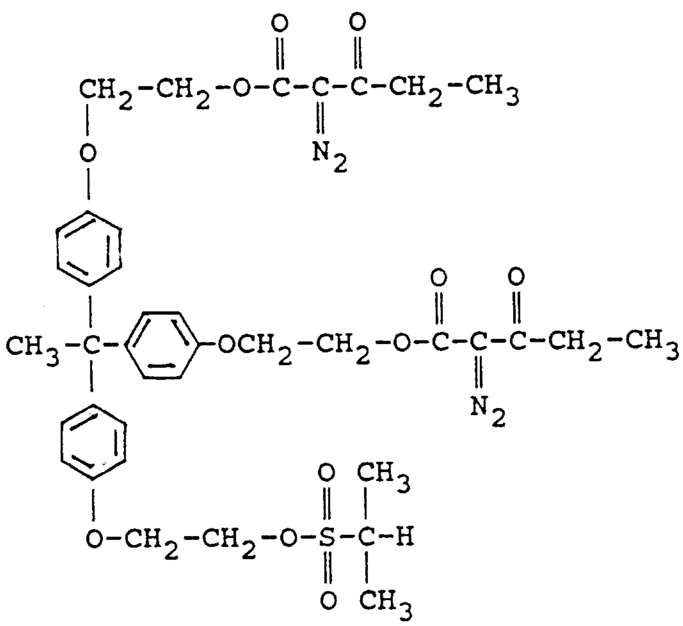

Beispiel 8, Verbindung 35

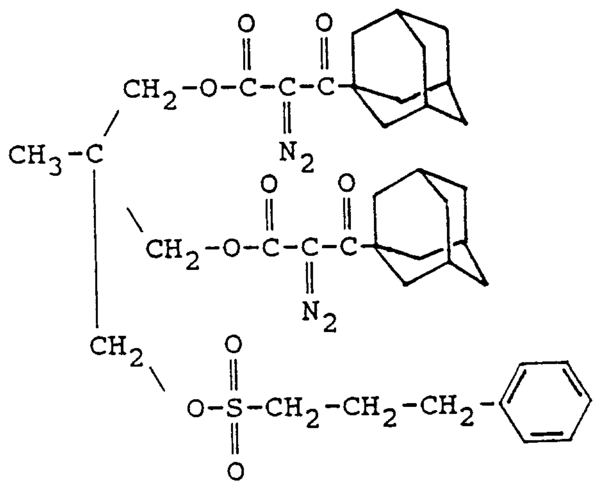

Beispiel 9, Verbindung 39

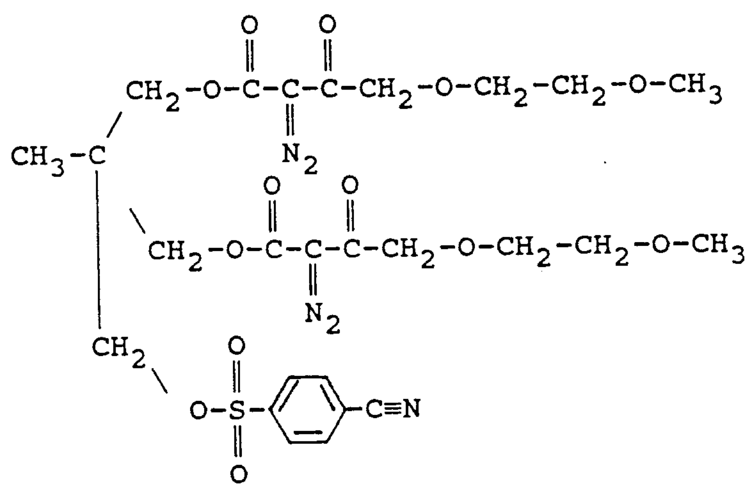

Beispiel 10, Verbindung 43

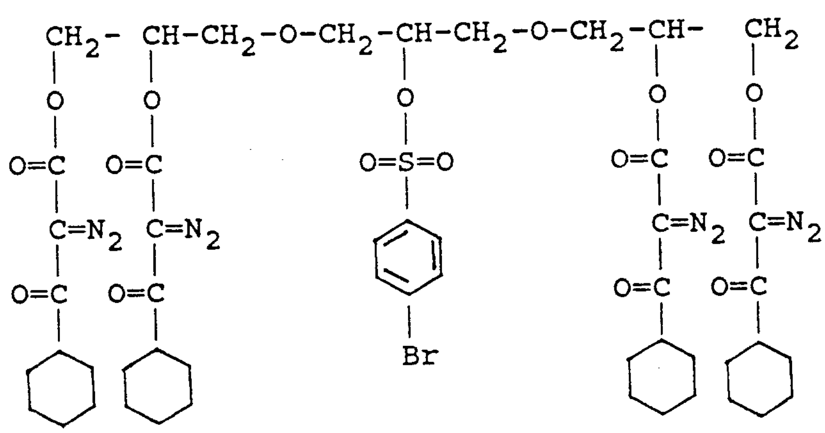

Beispiel 11
Verbindungen 48

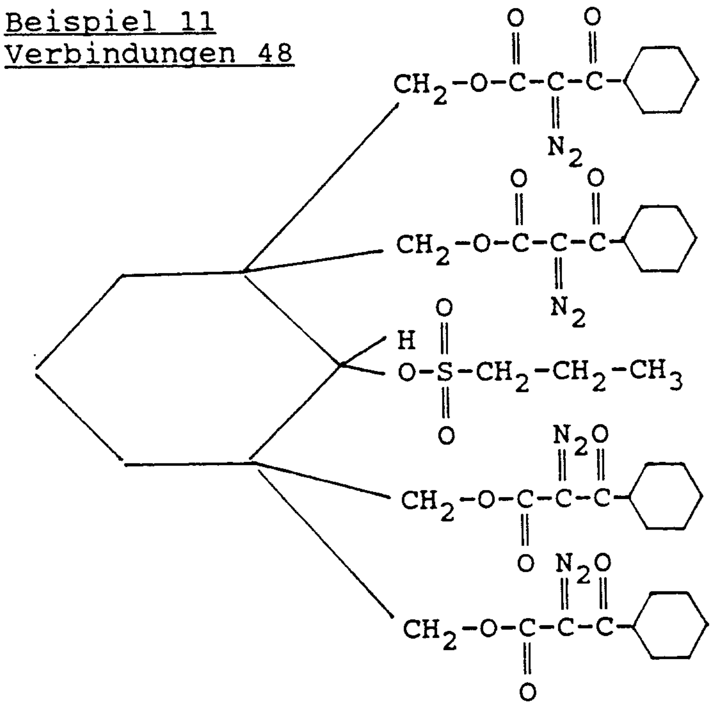

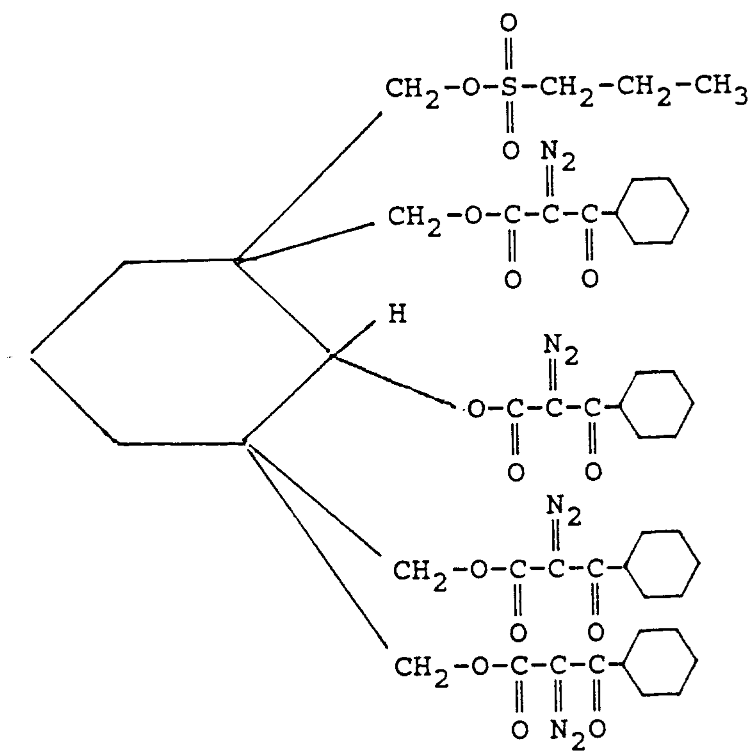

Isomerengemisch

Beispiel 12, Verbindung 52

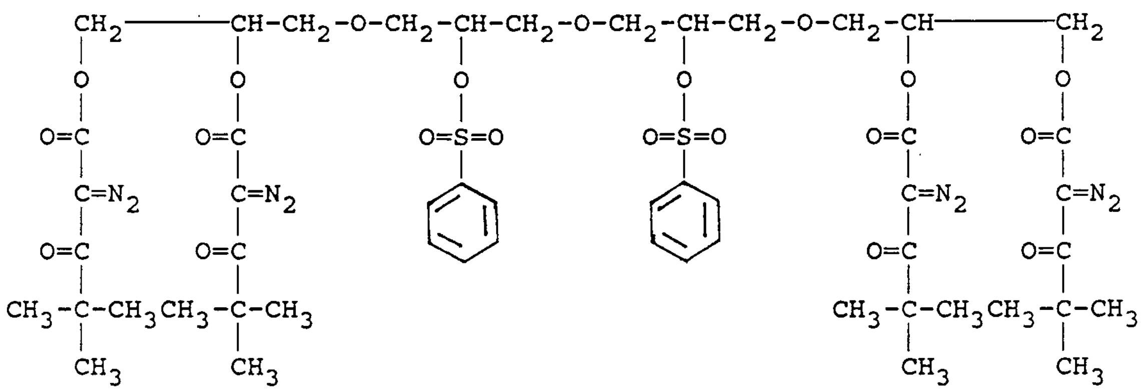

Beispiel 73 (Vergleichsbeispiel)

Analog zu Beispiel 9 (Stufe 4) werden 9,62 g (80 mmol) 2-(Hydroxymethyl)-2-methyl-1,3-propandiol zu einem trifunktionellen β-Ketoester (Verbindung **53**) umgesetzt, in diesem Fall aber mit 62,5 g (0,24 mol) 5-(3,6-Dioxa-1-hydroxyheptyliden)-2,2-dimethyl-1,3-dioxan-4,6-dion, so daß $R^1$ (wie in Beispiel 9) = $CH_2OCH_2CH_2OCH_3$ bedeutet, aber n = 0 ist.

Anschließend wird die Verbindung **53**, wie in Beispiel 9 (Stufe 5) beschrieben, mit 31,6 g (160 mmol) Tosylazid, 30,4 g (300 mmol) Triethylamin und 18,2 g (80 mmol) 4-Carboxyphenylsulfonylazid zu dem entsprechenden trifunktionellen α-Diazo-β-ketoester (**54**) der allgemeinen Formel I (n = 0) umgesetzt. Das Rohprodukt wird aus Ethanol umkristallisiert.

| | | | | |
|---|---|---|---|---|
| $C_{27}H_{38}N_6O_{15}$ (MG 686,6) | ber.:<br>gef.: | C 47,23 %<br>C 47,4 % | H 5,58 %<br>H 5,5 % | N 12,24 %<br>N 11,8 % |

Wie in der gleichzeitig eingereichten deutschen Patentanmeldung P 40 14 648.0 gezeigt wird, zeigen lichtempfindliche Gemische, die die Verbindung **54** gemäß Beispiel 73 als photoaktive Komponente enthalten, im Vergleich zu der erfindungsgemäßen Verbindung der allgemeinen Formel I zwar vergleichbare Ausbleicheigenschaften mit diesen, allerdings sind die bilddifferenzierenden Eigenschaften nicht zufriedenstellend.

## Patentansprüche

**1.** Mehrfunktionelle Verbindung mit α-Diazo-β-ketoester- und Sulfonsäureester-Einheiten gemäß der allgemeinen Formel I

$[R^1\text{-CO-C}(N_2)\text{-CO-O}]_{m-n}\text{-X-}[\text{O-SO}_2\text{-}R^2]_n$ (I)

worin

$R^1$ Ethyl, Isopropyl, ein gegebenenfalls substituierter aliphatischer Rest mit 4 bis 20 Kohlenstoffatomen oder ein gegebenenfalls substituierter cycloaliphatischer, araliphatischer oder aromatischer Rest mit 4 bis 20 Kohlenstoffatomen ist, in denen jeweils einzelne $CH_2$-Gruppen durch Sauerstoff- oder Schwefelatome, NH- und/oder CO-Gruppen ersetzt sein können und einzelne CH-Gruppen in einer aliphatischen Kette durch -N< ersetzt sein können,

$R^2$ ein gegebenenfalls substituierter aliphatischer Rest mit 1 bis 20 Kohlenstoffatomen oder ein gegebenenfalls substituierter cycloaliphatischer, araliphatischer oder aromatischer Rest mit 4 bis 20 Kohlenstoffatomen ist, in denen jeweils einzelne $CH_2$-Gruppen durch Sauerstoff- oder Schwefelatome, NH- und/oder CO-Gruppen und einzelne CH-Gruppen durch -N< ersetzt sein

können,

X ein gegebenenfalls substituierter aliphatischer, cycloaliphatischer, carbocyclischer, heterocyclischer oder araliphatischer Rest mit 2 bis 22 Kohlenstoffatomen ist, in dem einzelne $CH_2$-Gruppen durch Sauerstoff- oder Schwefelatome, $-NR^3-$ oder CO-Gruppen und einzelne CH-Gruppen durch -N< ersetzt sein können, worin

$R^3$ für Wasserstoff oder einen gegebenenfalls substituierten aliphatischen, carbocyclischen oder araliphatischen Rest steht,

m eine ganze Zahl von 3 bis 8 und

n eine ganze Zahl von 1 bis 3 ist, wobei $m-n \geqq 2$ ist.

**2.** Mehrfunktioneller Ester nach Anspruch 1, dadurch gekennzeichnet, daß $R^3$ Wasserstoff, $(C_1-C_3)$Alkyl, $(C_6-C_{12})$Aryl oder $(C_6-C_{11})$Aralkyl ist, wobei Aryl und Aralkyl gegebenenfalls am Kern durch $(C_1-C_3)$-Alkyl, $(C_1-C_3)$Alkoxy, Halogen, insbesondere Chlor oder Brom, oder Amino substituiert sein können.

**3.** Mehrfunktioneller Ester nach den Ansprüchen 1 oder 2, dadurch gekennzeichnet, daß $R^1$, $R^2$ und/oder X substituiert sind durch $(C_1-C_3)$Alkyl, $(C_1-C_3)$Alkoxy, Halogen, Amino oder Nitrilo, insbesondere durch $(C_1-C_3)$Alkyl oder $(C_1-C_3)$Alkoxy.

**4.** Mehrfunktioneller Ester nach einem oder mehreren der Ansprüche 1 bis 3, dadurch gekennzeichnet, daß sofern $R^1$ und/oder $R^2$ einen aliphatischen Rest darstellen und dieser substituiert ist, dieser 4 bis 10, Kettenglieder aufweist.

**5.** Mehrfunktioneller Ester nach Anspruch 4, dadurch gekennzeichnet, daß $CH_2$-Gruppen insbesondere durch Sauerstoffatome, -NH-Gruppen oder Ketogruppen ersetzt sind und diese Reste daher Ether-, Keto-, Ester-, Amido- und/oder Imidogruppen enthalten.

**6.** Mehrfunktioneller Ester nach einem oder mehreren der Ansprüche 1 bis 3, dadurch gekennzeichnet, daß sofern $R^1$ einen aliphatischen Rest darstellt und dieser unsubstituiert ist, dieser bis zu 20 Kettenglieder aufweist.

**7.** Mehrfunktioneller Ester nach einem oder mehreren der Ansprüche 1 bis 3, dadurch gekennzeichnet, daß $R^1$ und/oder $R^2$ ein cycloaliphatischer Rest bedeutet mit 4, 5, 6 oder 12, insbesondere 4, 5 oder 6, besonders bevorzugt 6, Ringgliedern, und der insbesondere unsubstituiert ist.

**8.** Mehrfunktioneller Ester nach einem oder mehreren der Ansprüche 1 bis 3, dadurch gekennzeichnet, daß $R^1$ und/oder $R^2$ ein araliphatischer Rest ist mit 1 bis 11, insbesondere 2 bis 5, Kettengliedern im aliphatischen Teil des Restes.

**9.** Mehrfunktioneller Ester nach Anspruch 8, dadurch gekennzeichnet, daß der aliphatische Teil des Restes $R^1$ und/oder $R^2$ 1 oder 2 Kettenglieder enthält, sofern dieser Teil eine reine Kohlenstoffkette aufweist.

**10.** Mehrfunktioneller Ester nach Anspruch 8, dadurch gekennzeichnet, daß der aliphatische Teil des Restes $R^1$ 3 bis 5 Kettenglieder aufweist, sofern in diesem Teil $CH_2$-Gruppen durch Heteroatome ersetzt sind.

**11.** Mehrfunktioneller Ester nach einem der Ansprüche 1 bis 10, dadurch gekennzeichnet, daß - sofern $R^2$ einen aliphatischen Rest darstellt, der unsubstituiert ist - dieser Rest 1 bis 8 Kettenglieder aufweist.

**12.** Mehrfunktioneller Ester nach einem oder mehreren der Ansprüche 1 bis 11, dadurch gekennzeichnet, daß im Rest X maximal 5, insbesondere maximal 3 $CH_2$-Gruppen durch die Heteroatome bzw. die genannten Gruppen ersetzt sind.

**13.** Mehrfunktioneller Ester nach einem oder mehreren der Ansprüche 1 bis 11, dadurch gekennzeichnet, daß der Rest X aliphatisch und unsubstituiert ist und maximal 6 Kohlenstoffatome aufweist.

**14.** Mehrfunktioneller Ester nach Anspruch 13, dadurch gekennzeichnet, daß dieser maximal eine C-C-Mehrfachbindung aufweist.

**15.** Mehrfunktioneller Ester nach Anspruch 12, dadurch gekennzeichnet, daß die $CH_2$-Gruppen ersetzende Heteroatome von einem Typ sind.

**16.** Mehrfunktioneller Ester nach Anspruch 12, dadurch gekennzeichnet, daß sofern eine CH-Gruppe durch

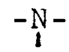

ersetzt ist, keine weitere Substitution im Rest X vorliegt.

**17.** Mehrfunktioneller Ester nach Anspruch 12, dadurch gekennzeichnet, daß X ein cycloaliphatischer Rest ist, in dem der cycloaliphatische Teil unsubstituiert ist und benachbart ist einer $CH_2$-Gruppe des aliphatischen Teils, die substituiert ist durch ein Heteroatom oder eine Gruppe des Anspruchs 1.

**18.** Mehrfunktioneller Ester nach Anspruch 17, dadurch gekennzeichnet, daß der cycloaliphatische Teil direkt benachbart ist zu einem Stickstoffatom, insbesondere der Gruppen

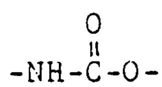 oder 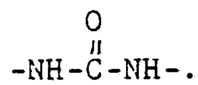.

**19.** Mehrfunktioneller Ester nach Anspruch 17, dadurch gekennzeichnet, daß der cycloaliphatische Teil über eine Ethylengruppe mit dem Sauerstoffatom eines

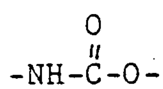

Restes verknüpft ist.

**20.** Mehrfunktioneller Ester nach Anspruch 12, dadurch gekennzeichnet, daß X ein araliphatischer Rest ist, wobei der aromatische Teil insbesondere einen Phenyl- bzw. Phenylenrest darstellt, der über ein Heteroatom mit dem aliphatischen Teil dieses Restes verknüpft ist.

**21.** Mehrfunktioneller Ester nach einem oder mehreren der Ansprüche 1 bis 20, dadurch gekennzeichnet, daß m eine ganze Zahl von 3 bis 8 und n eine ganze Zahl von 1 bis 3 ist.

**22.** Mehrfunktioneller Ester nach Anspruch 21, dadurch gekennzeichnet, daß m eine ganze Zahl von 3 bis 6 und n = 1 oder 2 ist.

**23.** Verfahren zur Herstellung mehrfunktioneller Ester nach einem oder mehreren der Ansprüche 1 bis 22, dadurch gekennzeichnet, daß ein $\beta$-Ketoester-Sulfonsäureester der allgemeinen Formel II in der 5- bis 50-fachen Menge eines geeigneten Lösungsmittels gelöst wird und dieser mit 1 bis 1,3 Äquivalenten eines Diazotransferreagenzes und einer Base, bevorzugt eines tertiären Amins, umgesetzt wird, das Reaktionsgemisch von überschüssigen Reagenzien und Lösungsmitteln befreit wird, in einem nicht mit Wasser mischbaren Lösungsmittel aufgenommen wird, mit Kalilauge und anschließend mit Wasser neutral gewaschen wird und nach dem Trocknen mit einem geeigneten Trocknungsmittel isoliert wird.

**24.** Verfahren nach Anspruch 23, dadurch gekennzeichnet, daß das Lösungsmittel einen Siedepunkt von 30° C bis 140° C aufweist und dieses auf -15° C bis +15° C gekühlt ist.

**25.** Verfahren nach den Ansprüchen 23 oder 24, dadurch gekennzeichnet, daß als Diazotransferreagenz ein aliphatisches oder aromatisches Sulfonylazid eingesetzt wird.

**26.** Verfahren nach einem oder mehreren der Ansprüche 23 bis 25, dadurch gekennzeichnet, daß nach vollendeter Zugabe aller Reagenzien die Mischung 5 bis 50 Minuten gerührt wird und anschließend für 1 bis 24 Stunden, bevorzugt für 2 bis 4 Stunden, bei Raumtemperatur gerührt wird.

**27.** Verfahren nach einem oder mehreren der Ansprüche 23 bis 26, dadurch gekennzeichnet, daß der $\beta$-Ketoester-Sulfonsäureester mit dem Diazotransferreagenz in dem Lösungsmittel vorgelegt wird und die Base anschließend hinzugefügt wird.

**28.** Verfahren nach einem oder mehreren der Ansprüche 23 bis 26, dadurch gekennzeichnet, daß der $\beta$-Ketoester-Sulfonsäureester mit der Base in dem Lösungsmittel vorgelöst wird und das Diazotransferreagenz hinzugefügt wird.

**29.** Verfahren nach einem oder mehreren der Ansprüche 23 bis 26, dadurch gekennzeichnet, daß der $\beta$-Ketoester-Sulfonsäureester zunächst mit 0,7 bis 0,9 Äquivalenten des Diazotransferreagenz versetzt wird, anschließend die Gesamtmenge der Base hinzugegeben wird, die Mischung auf Raumtemperatur erwärmt wird und nach dem Abkühlen mit 0,6 bis 0,1 Äquivalenten des Diazotransferreagenzes versetzt wird.

**30.** Verfahren nach einem oder mehrerer der Ansprüche 23 bis 29, dadurch gekennzeichnet, daß die Menge der zugesetzten Base äquimolar zu der Menge des Diazotransferreagenzes ist.

## Claims

**1.** A polyfunctional compound containing $\alpha$-diazo-$\beta$-keto ester units and sulfonate units, according to the formula I

$[R^1\text{-CO-C}(N_2)\text{-CO-O}]_{m-n}\text{-X-}[\text{O-SO}_2\text{-}R^2]_n$ (I)

in which

- $R^1$ is ethyl, isopropyl, an optionally substituted aliphatic radical having 4 to 20 carbon atoms or an optionally substituted cycloaliphatic, araliphatic or aromatic radical having 4 to 20 carbon atoms in which, in each case, individual $CH_2$ groups maybe replaced by oxygen or sulfur atoms, NH and/or CO groups, and individual CH groups in an aliphatic chain may be replaced by -N<,
- $R^2$ is an optionally substituted aliphatic radical having 1 to 20 carbon atoms or an optionally substituted cycloaliphatic, araliphatic or aromatic radical having 4 to 20 carbon atoms in which, in each case, individual $CH_2$ groups may be replaced by oxygen or sulfur atoms, NH and/or CO groups, and individual CH groups may be replaced by -N<,
- X is an optionally substituted aliphatic, cycloaliphatic, carbocyclic, heterocyclic or araliphatic radical having 2 to 22 carbon atoms in which individual $CH_2$ groups may be replaced by oxygen or sulfur atoms, -$NR^3$- or CO groups, and individual CH groups may be replaced by -N<, in which
- $R^3$ is hydrogen or an optionally substituted aliphatic, carbocyclic or araliphatic radical,
- m is an integer from 3 to 8 and
- n is an integer from 1 to 3, where m-n is $\geqq 2$.

**2.** A polyfunctional ester as claimed in claim 1, wherein $R^3$ is hydrogen, $(C_1\text{-}C_3)$alkyl, $(C_6\text{-}C_{12})$aryl or $(C_6\text{-}C_{11})$aralkyl, it being possible, if desired, for aryl and aralkyl to be substituted on the ring by $(C_1\text{-}C_3)$-alkyl, $(C_1\text{-}C_3)$alkoxy, halogen, in particular chlorine or bromine, or amino.

**3.** A polyfunctional ester as claimed in claim 1 or 2, wherein $R^1$, $R^2$ and/or X are substituted by $(C_1\text{-}C_3)$-alkyl, $(C_1\text{-}C_3)$alkoxy, halogen, amino or nitrile, in particular by $(C_1\text{-}C_3)$alkyl or $(C_1\text{-}C_3)$alkoxy.

**4.** A polyfunctional ester as claimed in one or more of claims 1 to 3, wherein, if $R^1$ and/or $R^2$ are aliphatic radicals and if these are substituted, they contain from 4 to 10 chain members.

**5.** A polyfunctional ester as claimed in claim 4, wherein $CH_2$ groups have been replaced, in particular by oxygen atoms, -NH- groups or keto groups, and these radicals therefore contain ether, keto, ester,

amido and/or imido groups.

6. A polyfunctional ester as claimed in one or more of claims 1 to 3, wherein, if $R^1$ is an aliphatic radical and if this is unsubstituted, it contains up to 20 chain members.

7. A polyfunctional ester as claimed in one or more of claims 1 to 3, wherein $R^1$ and/or $R^2$ are cycloaliphatic radicals having 4, 5, 6 or 12, in particular 4, 5 or 6, particularly preferably 6, ring members, and are, in particular, unsubstituted.

8. A polyfunctional ester as claimed in one or more of claims 1 to 3, wherein $R^1$ and/or $R^2$ are araliphatic radicals having 1 to 11, in particular 2 to 5, chain members in the aliphatic moiety of the radical.

9. A polyfunctional ester as claimed in claim 8, wherein the aliphatic moiety of the radical $R^1$ and/or $R^2$ contains 1 or 2 chain members if this moiety has a pure carbon chain.

10. A polyfunctional ester as claimed in claim 8, wherein the aliphatic moiety of the radical $R^1$ contains 3 to 5 chain members if $CH_2$ groups in this moiety have been replaced by heteroatoms.

11. A polyfunctional ester as claimed in any one of claims 1 to 10, wherein, if $R^2$ is an aliphatic radical which is unsubstituted, this radical contains 1 to 8 chain members.

12. A polyfunctional ester as claimed in one or more of claims 1 to 11, wherein a maximum of 5, in particular a maximum of 3, $CH_2$ groups in the radical X have been replaced by heteroatoms or said groups.

13. A polyfunctional ester as claimed in one or more of claims 1 to 11, wherein the radical X is aliphatic and unsubstituted and has a maximum of 6 carbon atoms.

14. A polyfunctional ester as claimed in claim 13, containing a maximum of one C-C multiple bond.

15. A polyfunctional ester as claimed in claim 12, wherein the heteroatoms replacing $CH_2$ groups are of one type.

16. A polyfunctional ester as claimed in claim 12, wherein, if a CH group has been replaced by -N-, no further substitution is present in the radical X.

17. A polyfunctional ester as claimed in claim 12, wherein X is a cycloaliphatic radical in which the cycloaliphatic moiety is unsubstituted and is adjacent to a $CH_2$ group of the aliphatic moiety which is substituted by a heteroatom or a group of claim 1.

18. A polyfunctional ester as claimed in claim 17, wherein the cycloaliphatic moiety is directly adjacent to a nitrogen atom, in particular of the group

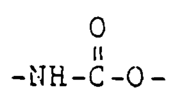 or 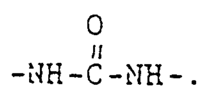

19. A polyfunctional ester as claimed in claim 17, wherein the cycloaliphatic moiety is linked to the oxygen atom of a

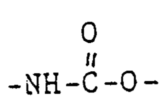

radical via an ethylene group.

**20.** A polyfunctional ester as claimed in claim 12, wherein X is an araliphatic radical in which the aromatic moiety is, in particular, a phenyl or phenylene radical which is linked to the aliphatic moiety of this radical via a heteroatom.

**21.** A polyfunctional ester as claimed in one or more of claims 1 to 20, wherein m is an integer from 3 to 8, and n is an integer from 1 to 3.

**22.** A polyfunctional ester as claimed in claim 21, wherein m is an integer from 3 to 6, and n is 1 or 2.

**23.** A process for the preparation of a polyfunctional ester as claimed in one or more of claims 1 to 22, which comprises dissolving a $\beta$-keto ester/sulfonate of the formula II in from 5 to 50 times the amount of a suitable solvent, and reacting it with 1 to 1.3 equivalents of a diazo transfer reagent and a base, preferably a tertiary amine, freeing the reaction mixture from excess reagents and solvents, taking it up in a water-immiscible solvent, washing the solution with potassium hydroxide solution and subsequently with water until neutral, and isolating the product after drying using a suitable desiccant.

**24.** The process as claimed in claim 23, wherein the solvent has a boiling point of from 30 °C to 140 °C and has been cooled to from -15 °C to +15 °C.

**25.** The process as claimed in either of claims 23 and 24, wherein the diazo transfer reagent employed is an aliphatic or aromatic sulfonyl azide.

**26.** The process as claimed in one or more of claims 23 to 25, wherein, after the addition of all the reagents is complete, the mixture is stirred for from 5 to 50 minutes and subsequently at room temperature for from 1 to 24 hours, preferably for from 2 to 4 hours.

**27.** The process as claimed in one or more of claims 23 to 26, wherein the $\beta$-keto ester/sulfonate is introduced into the solvent together with the diazo transfer reagent, and the base is subsequently added.

**28.** The process as claimed in one or more of claims 23 to 26, wherein the $\beta$-keto ester/sulfonate is predissolved in the solvent with the base, and the diazo transfer reagent is added.

**29.** The process as claimed in one or more of claims 23 to 26, wherein 0.7 to 0.9 equivalent of the diazo transfer reagent is initially added to the $\beta$-keto ester/sulfonate, all the base is subsequently added, the mixture is warmed to room temperature and, after cooling, 0.6 to 0.1 equivalent of the diazo transfer reagent is added.

**30.** The process as claimed in one or more of claims 23 to 29, wherein the amount of added base is equimolar to the amount of the diazo transfer reagent.

**Revendications**

**1.** Composé polyfonctionnel à groupements $\alpha$-diazo-$\beta$-cétoester et ester sulfonique, de formule générale I

$$[R^1\text{-CO-C}(N_2)\text{-CO-O}]_{m-n}\text{-X-}[\text{O-SO}_2\text{-}R^2]_n \qquad (I)$$

dans laquelle

$R^1$ est le groupe éthyle, isopropyle ou un radical aliphatique éventuellement substitué ayant de 4 à 20 atomes de carbone, ou un radical cycloaliphatique, araliphatique ou aromatique éventuellement substitué, ayant de 4 à 20 atomes de carbone, dans lequel des groupes $CH_2$ individuels peuvent être remplacés chacun par des atomes d'oxygène ou de soufre ou par des groupes NH et/ou CO, et des groupes CH individuels dans une chaîne aliphatique peuvent être remplacés chacun par $-N<$ ,

$R^2$ est un radical aliphatique éventuellement substitué ayant de 1 à 20 atomes de carbone, ou un radical cycloaliphatique, araliphatique ou aromatique éventuellement substitué, ayant de 4 à 20 atomes de carbone, dans lequel des groupes $CH_2$ individuels peuvent être remplacés chacun par des atomes d'oxygène ou de soufre ou par des groupes NH et/ou

CO, et des groupes CH individuels peuvent être remplacés chacun par -N<,

X représente un radical aliphatique, cycloaliphatique, carbocyclique, hétérocyclique ou araliphatique éventuellement substitué ayant de 2 à 22 atomes de carbone, dans lequel des groupes $CH_2$ individuels peuvent être remplacés par des atomes d'oxygène ou de soufre ou par le groupe $-NR^3-$ ou CO, et des groupes CH individuels peuvent être remplacés par -N<,

$R^3$ représentant un atome d'hydrogène ou un radical aliphatique, carbocyclique ou araliphatique éventuellement substitué,

m est un nombre entier allant de 3 à 8, et

n est un nombre entier allant de 1 à 3,

m - n étant ≧ 2.

**2.** Ester polyfonctionnel selon la revendication 1, caractérisé en ce que $R^3$ représente un atome d'hydrogène ou un alkyle en $C_1$-$C_3$, aryle en $C_6$-$C_{12}$ ou aralkyle en $C_6$-$C_{11}$, aryle et aralkyle pouvant éventuellement être substitués sur le noyau par des groupes alkyle en $C_1$-$C_3$, alcoxy en $C_1$-$C_3$, par des atomes d'halogène, en particulier de chlore ou de brome, ou par le groupe amino.

**3.** Ester polyfonctionnel selon la revendication 1 ou 2, caractérisé en ce que $R^1$, $R^2$ et/ou X sont substitués par des substituants alkyle en $C_1$-$C_3$, alcoxy en $C_1$-$C_3$, halogène, amino ou nitrilo, en particulier par un groupe alkyle en $C_1$-$C_3$ ou alcoxy en $C_1$-$C_3$.

**4.** Ester polyfonctionnel selon une ou plusieurs des revendications 1 à 3, caractérisé en ce que, lorsque $R^1$ et/ou $R^2$ représente(nt) un radical aliphatique et que celui-ci est substitué, il comporte de 4 à 10 chaînons.

**5.** Ester polyfonctionnel selon la revendication 4, caractérisé en ce que des groupes $CH_2$ sont remplacés en particulier par des atomes d'oxygène, des groupes -NH- ou des groupes céto, et ces radicaux contiennent par conséquent des groupes éther, céto, ester, amido et/ou imido.

**6.** Ester polyfonctionnel selon une ou plusieurs des revendications 1 à 3, caractérisé en ce que, lorsque $R^1$ représente un radical aliphatique et que celui-ci n'est pas substitué, il comporte jusqu'à 20 chaînons.

**7.** Ester polyfonctionnel selon une ou plusieurs des revendications 1 à 3, caractérisé en ce que $R^1$ et/ou $R^2$ représente(nt) un radical cycloaliphatique ayant 4, 5, 6 ou 12, en particulier 4, 5 ou 6, de préférence 6 chaînons formant le cycle, et qui est en particulier non substitué.

**8.** Ester polyfonctionnel selon une ou plusieurs des revendications 1 à 3, caractérisé en ce que $R^1$ et/ou $R^2$ est (sont) un radical araliphatique ayant de 1 à 11, en particulier de 2 à 5 chaînons dans le fragment aliphatique du radical.

**9.** Ester polyfonctionnel selon la revendication 8, caractérisé en ce que le fragment aliphatique du radical $R^1$ et/ou $R^2$ contient 1 ou 2 chaînons, dans la mesure où ce fragment comporte une chaîne purement carbonée.

**10.** Ester polyfonctionnel selon la revendication 8, caractérisé en ce que le fragment aliphatique du radical $R^1$ comporte de 3 à 5 chaînons, lorsque, dans ce fragment, des groupes $CH_2$ sont remplacés par des hétéroatomes.

**11.** Ester polyfonctionnel selon l'une des revendica tions 1 à 10, caractérisé en ce que - lorsque $R^2$ représente un radical aliphatique qui est non substitué - ce radical comporte de 1 à 8 chaînons.

**12.** Ester polyfonctionnel selon une ou plusieurs des revendications 1 à 11, caractérisé en ce que, dans le radical X, au maximum 5, en particulier au maximum 3 groupes $CH_2$ sont remplacés par les hétéroatomes ou par les groupes cités.

**13.** Ester polyfonctionnel selon une ou plusieurs des revendications 1 à 11, caractérisé en ce que le radical X est aliphatique et non substitué et comporte au maximum 6 atomes de carbone.

**14.** Ester polyfonctionnel selon la revendication 13, caractérisé en ce que celui-ci comporte au maximum une liaison multiple C-C.

**15.** Ester polyfonctionnel selon la revendication 12, caractérisé en ce que les hétéroatomes remplaçant des groupes $CH_2$ sont d'un seul type.

**16.** Ester polyfonctionnel selon la revendication 12, caractérisé en ce que, dans la mesure où un groupe CH est remplacé par

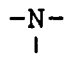,

aucun autre substituant n'est présent dans le radical X.

**17.** Ester polyfonctionnel selon la revendication 12, caractérisé en ce que X est un radical cycloaliphatique dans lequel le fragment cycloaliphatique n'est pas substitué et est voisin d'un groupe $CH_2$ du fragment aliphatique, qui est substitué par un hétéroatome ou par un groupe de la revendication 1.

**18.** Ester polyfonctionnel selon la revendication 17, caractérisé en ce que le fragment cycloaliphatique est immédiatement voisin d'un atome d'azote, en particulier du groupe

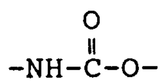 ou 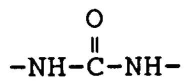.

**19.** Ester polyfonctionnel selon la revendication 17, caractérisé en ce que le fragment cycloaliphatique est lié par l'intermédiaire d'un groupe éthylène à l'atome d'oxygène d'un radical

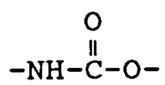.

**20.** Ester polyfonctionnel selon la revendication 12, caractérisé en ce que X est un radical araliphatique, le fragment aromatique représentant en particulier un reste phényle ou phénylène qui est lié par l'intermédiaire d'un hétéroatome au fragment aliphatique de ce radical.

**21.** Ester polyfonctionnel selon une ou plusieurs des revendications 1 à 20, caractérisé en ce que m est un nombre entier allant de 3 à 8 et n est un nombre entier allant de 1 à 3.

**22.** Ester polyfonctionnel selon la revendication 21, caractérisé en ce que m est un nombre entier allant de 3 à 6 et n = 1 ou 2.

**23.** Procédé pour la préparation d'esters polyfonctionnels selon une ou plusieurs des revendications 1 à 22, caractérisé en ce que l'on dissout un $\beta$-cétoester-ester sulfonique de formule générale II dans 5 à 50 fois sa quantité d'un solvant approprié et on le fait réagir avec 1 à 1,3 équivalent d'un réactif de transfert de fonction diazo et d'une base, de préférence d'une amine tertiaire, on sépare le mélange réactionnel des solvants et réactifs en excès, on le reprend dans un solvant non miscible à l'eau, on le lave avec une solution d'hydroxyde de potassium et ensuite à neutralité avec de l'eau, et, après séchage à l'aide d'un desséchant approprié, on l'isole.

**24.** Procédé selon la revendication 23, caractérisé en ce que le solvant présente un point d'ébullition de 30 à 140 °C et est refroidi jusqu'à -15 à +15 °C.

**25.** Procédé selon la revendication 23 ou 24, caractérisé en ce que, en tant que réactif de transfert de fonction diazo, on utilise un sulfonylazide aliphatique ou aromatique.

**26.** Procédé selon une ou plusieurs des revendications 23 à 25, caractérisé en ce que, une fois terminée l'addition de tous les réactifs, on agite le mélange pendant 5 à 50 minutes et on l'agite ensuite à la température ambiante pendant 1 à 24 heures, de préférence pendant 2 à 4 heures.

**27.** Procédé selon une ou plusieurs des revendications 23 à 26, caractérisé en ce que l'on dispose au préalable dans le solvant le $\beta$-cétoester-ester sulfonique avec le réactif de transfert de fonction diazo, et on y ajoute ensuite la base.

**28.** Procédé selon une ou plusieurs des revendications 23 à 26, caractérisé en ce que l'on dissout au préalable dans le solvant le $\beta$-cétoester-ester sulfonique avec la base et on y ajoute le réactif de transfert de fonction diazo.

**29.** Procédé selon une ou plusieurs des revendications 23 à 26, caractérisé en ce que l'on ajoute d'abord au $\beta$-cétoester-ester sulfonique 0,7 à 0,9 équivalent du réactif de transfert de fonction diazo, puis on y ajoute la quantité totale de la base, on porte le mélange à la température ambiante et, après refroidissement, on y ajoute 0,6 à 0,1 équivalent du réactif de transfert de fonction diazo.

**30.** Procédé selon une ou plusieurs des revendications 23 à29, caractérisé en ce que la quantité de la base ajoutée est équimolaire par rapport à la quantité du réactif de transfert de fonction diazo.